# EUROPEAN PATENT APPLICATION

(11) **EP 2 602 330 A1**
(43) Date of publication of application: **12.06.2013**
(21) Application number: 11192330.6
(22) Date of filing: 07.12.2011
(51) Int. Cl.: C12Q 1/68

(54) **Method of determination of cancer cell drug sensitivity towards Aurora kinase inhibitors and overcoming their resistance**

(71) Applicant: Palacky University, Olomouc, 77147 Olomouc (CZ); Institute of Animal Physiology and Genetics ASCR, v.v.i., 27721 Libechov (CZ)
(72) Inventor: Kollareddy, Madhusudhan Reddy, 524137 Andhra Pradesh (IN); Hajduch, Marian, 79305 Moravsky Beroun (CZ); Dzubak, Petr, 77900 Olomouc - Hejcin (CZ); Srovnal, Josef, 77900 Olomouc - Nova Ulice (CZ); Hrabakova, Rita, 41801 Bilina (CZ); Kovarova, Hana, 53002 Pardubice (CZ)
(74) Representative: Gabrielova, Marta

(57) **Abstract**

The present invention relates to a method for determining the sensitivity and/or resistance of a patient suffering from a cancer disease to Aurora kinase inhibitor therapy, which comprises determining *in vitro* in the cancer cells or body fluids taken from the patient the expression of at least one gene selected from a particular group and/or determining *in vitro* in the cancer cells or body fluids taken from the patient the level of at least one protein selected from a particular group. Also, the invention relates to a group of compounds which allow overcoming the resistance.

## Description

### Field of Art

The invention relates to a method of determination of a cancer cell drug sensitivity (i.e., whether the cancer cell is sensitive or resistant) towards Aurora kinase inhibitors as well as to a compound which can be used for overcoming the resistance.

### Background Art

Chemotherapy is one the main forms of treatment in patients with malignant cancers. Even though cancer patients respond to a particular drug initially, during the long-term treatment the relapse is common. Selection pressure on cancer cells, make them to evolve with better genotypes to evade the drug induced cell death. The drug resistance is one of the major obstacles in cancer chemotherapy (Gottesman M.M. et al., Annual Review of Medicine 2002; 53, 615-27). In order to tackle the problem of drug resistance, identification and understanding of cancer cell resistance mechanisms towards a particular drug is necessary. Some of the common drug resistance mechanisms include up-regulation of drug transporters (Parekh M. et al., Biomedical Pharmacology 1997; 56, 461-70) mutation of the drug target (Gorre M.E. Science 2001; 293, 876-70) up-regulation of CYP450 (McFayden M.C.E. et al., British Journal of Cancer 2004; 91, 966-71) amplification of drug target (Gorre M.E. et al., Science 2001; 293, 876-70) and many others. Cancer drug resistance mechanisms are very complex and more than one resistance mechanism may prevail to a particular drug. The drug resistance is not mediated by one gene; rather it is the consequence of many gene effects. Studies on drug resistance mechanisms in parallel with preclinical studies yields much information, which can be applied in early clinical trial studies to predict the response. Recently Aurora kinases (A, B, and C/serine threonine kinases) gained much attention due to their implication in several types of cancers. Aurora kinases are involved in multiple functions in mitosis. Aurora A is involved in mitotic entry, separation of centriole pairs, accurate bipolar spindle assembly, alignment of metaphase chromosomes and completion of cytokinesis (Marumoto T. et al., The Journal of Biological Chemistry 2004; 278, 51786-95). Aurora B is a chromosomal passenger protein involved in the regulation of chromosomal bi-orientation, and regulating the association between kinetochores and microtubules, and cytokinesis (Adams R.R. et al., The Journal of Biological Chemistry 2001; 15, 865-80). Aurora C exhibits similar functions to those assigned to Aurora B and is required for cytokinesis. The above mentioned functions are directly involved in maintaining genomic stability. The relation between Aurora kinases overexpression and transformation has been reported in many cancers. Aurora A was shown to overexpress in colorectal, renal, melanoma, and breast cancers (Bischoff J.R. et al., EMBO Journal 1998; 17, 3052-65). Mainly Aurora B was shown to overexpress in colorectal cancer (Katayama H. et al., Journal of National Cancer Institute 1999; 91, 1160-62). Aurora B was also implicated in thyroid anaplastic carcinoma (Sorrentino R. et al., Journal of Clinical Endocrinology and Metabolism 2004; 90, 928-35) and glioblastoma (Zeng W.F. et al., Journal of Clinical Pathology 2007; 60, 218-21). Apart from this, Aurora kinases were shown to overexpress in many other advanced solid carcinomas. Aurora kinases overexpression in many solid cancers is the basis of strong rational to discover and develop several Aurora kinase inhibitors. Some Aurora kinase inhibitors are already in the clinical trials and have shown promising anticancer activity in advanced solid cancers. AZD1152 (AstraZeneca) is currently in phase II studies and have proven effective in colon and melanoma cancers. It achieved stable diseases in progressive cancers (Schellens J.H. et al., Journal of Clinical Oncology 2006; 24, 3008 (Suppl)). Similarly AT-9283 (Astex) (Kristeleit R. et al., ASCO Annual Meeting 2009), PHA-739358 (Pfizer) (Paquette R. et al., Haemotology Meeting Reports 2008; 2, 92-93), and MLN8237 (Milliennium) (Infante J. et al., European Journal of Cancer Supplements 2008; 6, 90-91), MLN8054 (Milliennium) (Dees E.C. et al., Cancer Chemotherapy and Phramacology 2011; 67, 945-54), VX-680 (Vertex) (Bebbington D. et al., Bioorganic & medicical chemistry letters 2009; 19, 3586-92) were proven to be very promising in the clinical trials.

CYC 116 (4-methyl-5-(2-(4-morpholinophenylamino)pyrimidin-4-yl)thiazol-2-amine), discovered and developed by Cyclacel pharmaceuticals (Dundee, UK) is a novel pan-Aurora kinase inhibitor. It showed promising anticancer activity in both preclinical (Wang S. et al., Journal of Medicinal Chemistry 2010; 53, 4367-78) and early clinical studies. Apart from Aurora kinases, (Aurora A - 44 nM, Aurora B - 19 nM, Aurora C- 65 nM) CYC116 also inhibits other oncogenic kinases including VEGFR2 and Flt-3. ZM447439 (N-[4-[[6-Methoxy-7-3-(4-morpholinyl)propoxy]4quinazolinyl]amino]phenyl]-benzamide), is a first generation Aurora kinase inhibitor.

The present invention provides a group of genes the expression of which or the level of proteins coded by the genes changes with the resistance towards Aurora kinase inhibitors. Therefore, the present invention provides a method for determining the sensitivity of a patient suffering from a cancer disease to Aurora kinase inhibitor therapy and therapeutic approaches to overcome these drug resistance mechanisms.

### Disclosure of the Invention

The object of the invention is a method for determining the sensitivity of a patient suffering from a cancer disease to Aurora kinase inhibitor therapy, which comprises determining in *vitro* in the cancer cells taken from the patient the expression or copy number changes of at least one gene selected from the group comprising:

| **Gene** | **Change in expression determining resistance** | **Average Fold change in expression determining resistance** | **Copy number changes** |
|---|---|---|---|
| PBX1 | increase | 3 | |
| **MID1** | **decrease** | **-10** | Deletion in p53-/-: CYC116 clones |
| **EHF** | **increase** | **7** | |
| ALDH3A1 | increase | 2 | Deletion in one p53-/-: CYC116 clone |
| SSFA2 | decrease | -2 | Deletion in two p53-/-: CYC116 clones |
| SEPT2 | decrease | -2 | |
| PVRL3 | decrease | -2 | Amplification in one p53-/-: CYC116 clone |
| SYTL2 | increase | 4 | Amplification in one p53+/+: CYC116 clone Amplification in all p53-/-: CYC116 clones |
| KLK7 | increase | 2 | Amplification in one p53+/+: CYC116 clone |
| **ARHGAP29** | **decrease** | **-5** | |
| **A4GALT** | **increase** | **3** | Amplification in one p53+/+: CYC116 clone |
| APOBEC3H | increase | 2.3 | |
| OAS1 | increase | 1.4 | |
| 8084630 | increase | 3 | Amplification in one p53+/+: CYC116 clone Amplification in one p53-/-: CYC116 clone |
| FXYD3 | increase | 3 | |
| TSPAN5 | decrease | -3 | Deletion in all p53-/-: CYC116 clones |
| **CYP1A1** | **increase** | **5.3** | Amplification in one p53-/-: CYC116 clone |
| **PRKACB** | **decrease** | **-6** | Amplification in all p53-/-: CYC116 clones |
| AVP!1 | increase | 2 | Amplification in one p53+/+: CYC116 |
| | | | clone Amplification in all p53-/-: CYC116 clones |
| IGF2BP3 | decrease | -2 | Amplification in two p53+/+: CYC116 clones Amplification in one p53-/-: CYC116 clones |
| **GJC1** | **decrease** | **-4** | Amplification in two p53-/-: CYC116 clones |
| NRP2 | increase | 2 | Amplification in one p53-/-: CYC116 clone |
| HAS2 | increase | 2.1 | Deletion in two p53-/-: CYC116 clone |
| SCG2 | decrease | -1.4 | Amplification in one p53-/-: CYC116 clone |
| AQ P3 | increase | 2 | |
| FRMD5 | decrease | -2.2 | Amplification in two p53-/-: CYC116 clones |
| IF144 | increase | 2.3 | |
| SPRY4 | decrease | -2 | |
| RNF125 | increase | 2 | Amplification in all p53-/-: CYC116 clones |
| ZFP36L1 | increase | 1.2 | Deletion in one p53+/+: CYC116 clones Amplification in one p53-/-: CYC116 clone |
| AREG | increase | 2 | Amplification in all p53-/-: CYC116 clones |
| PRSS22 | increase | 1.4 | Amplification in one p53+/+: CYC116 clone Amplification in two p53-/-: CYC116 clones |
| FNTA | decrease | -2 | |
| ABCC2 | decrease | -3.1 | Amplification in one p53-/-: CYC116 clone |
| SERINC5 | increase | 2.3 | Amplification in two p53-/-: CYC116 clones |
| NEK10 | increase | 1.3 | Deletion in one p53-/-: CYC116 clone |
| NOV | increase | 1.4 | |
| GRHL3 | increase | 1.3 | |
| NEK3 | decrease | -2.3 | |
| KLK8 | increase | 1.4 | Amplification in one p53+/+: CYC116 clone |
| ELOVL6 | decrease | -2.1 | Deletion in all p53-/-: CYC116 clones |
| 8062284 | increase | 2.1 | Amplification in one p53+/+: CYC116 clone Amplification in one p53-/-: CYC116 clone |
| FYTTD1 | decrease | -1.6 | Amplification in one p53+/+: CYC116 clone Amplification in two p53-/-: CYC116 clones |
| PRKCQ | increase | 1.7 | Amplification in two p53-/-: CYC116 clones |
| ATP9A | increase | 1.5 | |
| DFNA5 | decrease | -2 | Amplification in two p53+/+: CYC116 clones |
| PTK6 | increase | 1.4 | Amplification in two p53+/+: CYC116 clones Amplification in one p53-/-: CYC116 clone |
| SYK | increase | 1.6 | Deletion in two p53-/-: CYC116 clones |
| ALDH1A3 | increase | 2.1 | |
| APOBEC3F | increase | 2.4 | Amplification in one p53+/+: CYC116 clone |
| CYP4F12 | increase | 2.1 | |
| MAML2 | increase | 2.4 | Amplification in two p53-/-: CYC116 clones |
| SLC37A2 | increase | 2 | Amplification in two p53+/+: CYC116 |
| | | | clones Amplification in all p53-/-: CYC116 clones |
| PAAF1 | increase | 1.6 | Amplification in one p53+/+: CYC116 clone Amplification in all p53-/-: CYC116 clones |
| NEBL | decrease | -1.4 | Deletion in one p53-/-: CYC116 clone Amplification in two p53-/-: CYC116 clone |
| CYP4F3 | increase | 2 | |
| GNG5 | decrease | -1.6 | |
| KLK6 | increase | 2.1 | Amplification in one p53+/+: CYC116 clone |
| ITGB7 | increase | 3 | |
| NHS | increase | 1.2 | Amplification in two p53-/-: CYC116 clones |
| ATP13A3 | increase | 1.1 | Amplification in one p53-/-: CYC116 clone |
| SLC2A1 | increase | 1.7 | |
| INTS10 | decrease | -1.3 | Deletion in all p53-/-: CYC116 clones |
| HOXA2 | increase | 1.4 | Amplification in one p53+/+: CYC116 clone Amplification in one p53-/-: CYC116 clone |
| ANKH | increase | 1.4 | |
| SOX4 | decrease | -1.4 | Amplification in all p53-/-: CYC116 clones |
| M F12 | increase | 1.6 | Amplification in one p53-/-: CYC116 clone |
| HOXB9 | increase | 2.4 | Amplification in one p53-/-: CYC116 clone |
| KLK10 | increase | 2.9 | Amplification in one p53+/+: CYC116 clone |
| KRTAP3 | increase | 1.3 | Amplification in one p53+/+: CYC116 clone Amplification in one p53-/-: CYC116 clone |
| C21orf63 | increase | 1.4 | Amplification in two p53+/+: CYC116 clones |
| APOBEC3C | increase | 2.4 | Amplification in one p53+/+: CYC116 clone |
| FAM49A | increase | 1.3 | Deletion in two p53-/-: CYC116 clones |
| TRAF3lPl | decrease | -1.2 | Deletion in two p53-/-: CYC116 clones |
| S100A14 | decrease | -2 | Amplification in one p53-/-: CYC116 clone |
| C3orf57 | increase | 1.9 | Amplification in one p53-/-: CYC116 clone |
| LTBP3 | increase | 1.5 | Amplification in one p53+/+: CYC116 clone Amplification in all p53-/-: CYC116 clone |
| CTSC | increase | 1.5 | Amplification in one p53+/+: CYC116 clone Amplification in two p53-/-: CYC116 clone |
| LOXL4 | increase | 1.2 | Amplification in two p53-/-: CYC116 clones |
| HAS3 | increase | 1.8 | Amplification in one p53+/+: CYC116 clone Amplification in two p53-/-: CYC116 clones |
| TRIM16L | decrease | -1.3 | Deletion in two p53-/-: CYC116 clones |
| PDE7A | decrease | -1.5 | Deletion in all p53-/-: CYC116 clones |
| RAB27B | increase | 2.2 | Amplification in two p53-/-: CYC116 clone Deletion in one p53-/-: CYC116 clone |
| IL13RA2 | increase | 1.6 | |
| ETS2 | decrease | -1.2 | Amplification in one p53+/+: CYC116 clone |
| RPL30 | decrease | -1.4 | |
| CR2 | increase | 2.4 | Deletion in one p53-/-: CYC116 clone |
| LPIN1 | decrease | -1.9 | Deletion in two p53-/-: CYC116 clones |
| **BCL2L1** | **increase** | **1.6** | Amplification in two p53+/+: CYC116 clone |
| PERP | increase | 1.6 | |
| HDAC2 | decrease | -1.3 | Amplification in two p53-/-: CYC116 clones |
| PORCN | increase | 1.4 | Amplification in one p53+/+: CYC116 clone Amplification in all p53-/-: CYC116 clone |
| SECTM1 | increase | 1.6 | |
| HSP90AB3P | decrease | -1.3 | |
| HSP90AB1 | decrease | -1.3 | |
| RPP30 | decrease | -1.3 | Amplification in one p53-/-: CYC116 clones |
| PKIB | decrease | -1.8 | Deletion in one p53+/+: CYC116 clone Amplification in all p53-/-: CYC116 clone |
| IGFBP6 | increase | 2.3 | |
| SAMD13 | decrease | -2.1 | |
| MAL2 | decrease | -23 | |
| SOLE | decrease | -4 | |
| CD33 | increase | 2.2 | Deletion in one p53+/+: ZM clone Amplification in two p53-/-: ZM clones |
| ZNF84 | decrease | -1.4 | |
| WLS | increase | 2 | |
| SYTL5 | decrease | -2.9 | |
| SLC7A8 | increase | 2.5 | Amplification in two p53-/-: CYC116 clones Amplification in two p53-/-: ZM clones |
| PPFIBP1 | decrease | -1.5 | |
| ZNF493 | decrease | -1.7 | |
| SLC5A1 | increase | 1.5 | |
| STXBP6 | decrease | -1.2 | Amplification in all p53-/-: CYC116 clones Amplification in all p53-/-: ZM clones |
| ZNF675 | decrease | -1.7 | |
| 8099393 | decrease | -1.4 | Amplification in one p53-/-: CYC116 clone Amplification in one p53-/-: ZM clone |
| BAMBI | increase | 1.8 | |
| AMOTL1 | decrease | -1.2 | |
| CLU | decrease | -1.4 | Deletion in one p53+/+: CYC116 clone |
| ZNF26 | decrease | -2.3 | |
| ZNF91 | decrease | -2.1 | Amplification in one p53-/-: ZM clone |
| ZNF266 | decrease | -2.5 | |
| IL18 | decrease | -1.5 | Amplification in all p53-/-: CYC116 clones |
| **FAM122B** | **decrease** | **-1.7** | Deletion in one p53+/+: ZM clone |
| DOCK5 | decrease | -1.3 | Deletion in all p53-/-: CYC116 clones |
| SLC04A1 | increase | 1.7 | Amplification in one p53-/-: CYC116 clone Amplification in one p53-/-: ZM clone |
| SNORD5 | decrease | -1.8 | Amplification in all p53-/-: CYC116 clones |
| SNORA18 | decrease | -1.8 | Amplification in all p53-/-: CYC116 clones |
| MIR1304 | decrease | -1.8 | Amplification in all p53-/-: CYC116 clones |
| ILF2 | decrease | -1.8 | |
| ATP6AP1 L | increase | 1.6 | Amplification in all p53-/-: CYC116 clones |
| MEF2C | decrease | -2 | Amplification in all p53-/-: CYC116 clones |
| C5orf13 | increase | 1.1 | Amplification in all p53-/-: CYC116 clones Amplification in one p53-/-: ZM clone |
| EXOSC9 | decrease | -1.6 | Deletion in all p53-/-: CYC116 clones |
| ALDH2 | increase | 1.6 | Amplification in one p53+/+: CYC116 clone Amplification in one p53-/-: ZM clone |
| FUT8 | decrease | -1.2 | |
| CDA | increase | 1.1 | Amplification in one p53+/+: CYC116 clone |
| TOX2 | increase | 1.6 | Deletion in one p53+/+: ZM clone |
| FGF9 | increase | 1.7 | |
| OAS3 | increase | 1.5 | |
| SEMA3D | increase | 1.8 | Amplification in one p53-/-: CYC116 clone |
| MIR15A | decrease | -2.2 | Deletion in all p53-/-: CYC116 clones |
| DLEU2 | decrease | -2.1 | Deletion in all p53-/-: CYC116 clones |
| MIR16-1 | decrease | -2.2 | Deletion in all p53-/-: CYC116 clones |
| USP22 | increase | 1.8 | |
| **INPP4B** | **decrease** | **-2.2** | Deletion in all p53-/-: CYC116 clones |
| TNS4 | increase | 1.86 | Amplification in two p53-/-: ZM clones |
| MNS1 | decrease | -2.7 | |
| 7893924 | increase | 38.3 | |
| TCF21 | decrease | -2 | Deletion in one p53+/+: CYC116 clone |
| ZBED2 | decrease | -1.5 | Amplification in two p53+/+: ZM clones |
| C1 DP1 | decrease | -1.5 | |
| **BDNF** | **decrease** | **-2** | Deletion in all p53+/+: ZM clones |
| **PPAP2B** | **increase** | **1.4** | Amplification in one p53+/+: ZM clone |
| 7894891 | increase | 3.4 | |
| CDC23 | decrease | -1.6 | Deletion in one p53+/+: ZM clone |
| 8109424 | increase | 2.6 | |
| SMNDC1 | decrease | -1.5 | |
| SART3 | decrease | -1.4 | |
| DDX5 | decrease | -1.7 | |
| MMP14 | decrease | -1.4 | Deletion in two p53+/+: CYC116 clones |
| FANCL | decrease | -1.6 | Deletion in two p53-/-: CYC116 clones Amplification in one p53+/+: ZM clone |
| 8098287 | decrease | -2.1 | Deletion in one p53+/+: CYC116 clone |
| TARDBP | decrease | -1.7 | |
| CASP4 | increase | 1.4 | Amplification in one p53+/+: ZM clone |
| SNORD22 | decrease | -1.6 | Amplification in all p53-/-: CYC116 clone Amplification in one p53+/+: ZM clone |
| SNORD28 | decrease | -1.6 | Amplification in all p53-/-: CYC116 clone Amplification in one p53+/+: ZM clone |
| SNORD29 | decrease | -1.6 | Amplification in all p53-/-: CYC116 clone Amplification in one p53+/+: ZM clone |
| SNORD30 | decrease | -1.6 | Amplification in all p53-/-: CYC116 clone Amplification in one p53+/+: ZM clone |
| **ERI1** | **decrease** | **-2.1** | Deletion in all p53-/-: CYC116 clones |
| RPSA | decrease | -1.2 | Deletion in one p53-/-: CYC116 clone |
| CPOX | decrease | -1.6 | Amplification in one p53+/+: ZM clone |
| 7894781 | decrease | -1.5 | |
| PALLD | decrease | -3.5 | Deletion in one p53+/+: CYC116 clone Deletion in all p53-/-: CYC116 clones Amplification in one p53+/+: ZM clone |
| MKX | decrease | -2.5 | Amplification in one p53+/+: ZM clone |
| CSMD3 | increase | 2 | Deletion in one p53-/-: CYC116 clone Amplification in one p53+/+: ZM clone |
| ENC1 | decrease | -2.6 | Amplification in all p53-/-: CYC116 clones Amplification in one p53+/+: ZM clone |
| **SERINC2** | **increase** | **2.8** | Amplification in one p53+/+: CYC116 |
| | | | clone Deletion in one p53+/+: ZM clone |
| CID | decrease | -1.4 | |
| CAV1 | decrease | -2.6 | Amplification in two p53+/+: CYC116 clones |
| AKT3 | increase | 2.2 | Amplification in one p53+/+: CYC116 clone Deletion in one p53-/-: CYC116 clone Amplification in one p53+/+: ZM clone Deletion in one p53+/+: ZM clone |
| **ANXA10** | **decrease** | **-2.4** | Amplification in one p53+/+: ZM clone |
| KLRC2 | decrease | -2.7 | Amplification in one p53+/+: CYC116 clone Amplification in one p53+/+: ZM clone |
| **CYP24A1** | **decrease** | **-38.7** | |
| WNT16 | decrease | -1.9 | Amplification in two p53+/+: CYC116 clones |
| 8148309 | decrease | -4 | Deletion in one p53-/-: CYC116 clone |
| **CAMK2D** | **decrease** | **-2.5** | Deletion in all p53-/-: CYC116 clones Deletion in one p53-/-: ZM clone |
| RHOBTB3 | decrease | -1.9 | Amplification in all p53-/-: CYC116 clones |
| PDE4B | decrease | -3 | Amplification in one p53-/-: CYC116 clone |
| **HTR7** | **decrease** | **-2.1** | Amplification in two p53-/-: CYC116 clones |
| COL12A1 | decrease | -1.8 | Deletion in one p53+/+: CYC116 clone Amplification in all p53-/-: CYC116 clones Amplification in one p53-/-: ZM clone |
| TIAM1 | decrease | -1.5 | Amplification in one p53+/+: CYC116 clone |
| KLRC3 | decrease | -2.2 | Amplification in one p53+/+: CYC116 clone |
| KRT23 | decrease | -1 | Amplification in one p53+/+: CYC116 clone Amplification in one p53-/-: CYC116 clone Amplification in two p53-/-: ZM clones |
| ZNF280A | decrease | -1.7 | Amplification in one p53+/+: CYC116 clone |
| **TBX3** | **increase** | **2.2** | Amplification in one p53+/+: CYC116 clone Deletion in one p53-/-: CYC116 clone Deletion in all p53-/-: ZM clones |
| UNC13A | increase | 1.3 | Amplification in one p53+/+: CYC116 clone Amplification in two p53-/-: CYC116 clones Amplification in two p53-/-: ZM clones |
| RUNX2 | increase | 2 | Amplification in two p53-/-: CYC116 clones |
| TRIB2 | increase | 1.6 | Deletion in two p53-/-: CYC116 clones |
| **TSPAN1** | **increase** | **2.5** | Amplification in one p53+/+: CYC116 clone Deletion in one p53+/+: ZM clone |
| ARMC4 | decrease | -3.5 | Amplification in one p53+/+: ZM clone |
| MPP7 | decrease | -2.6 | Deletion in two p53-/-: CYC116 clones Amplification in one p53+/+: ZM clone Deletion in all p53-/-: ZM clones |
| **KRT7** | **increase** | **2** | |

The increase and decrease of the expression of the genes in the cancer cells in comparison to the expression in controls as shown in the table indicates the resistance of the cancer towards Aurora kinase inhibitors. The p-value is in the range of 1.14x10⁻¹¹ - 0.0009. Corresponding cytogenetic changes were also presented as a gene copy number alterations.

The controls to which the drug resistant cancer cells are compared are usually their genetically identical drug sensitive counterparts. For validation study on tumor patient primary tumors, cells directly isolated from untreated patient tumors were tested for in vitro drug response. The nucleic acids isolated from the most sensitive versus the most resistant patient tumors were used for validation of gene expression signatures identified previously in cell line experiments.

The increase or decrease, respectively, in the expression of the genes listed herein was observed in several tested cancer cell lines resistant to Aurora kinase inhibitors. Therefore, the changes in the expression of the genes are indicative of resistance towards Aurora kinase inhibitors.

The expression can be determined at the RNA level or at the protein level.

Preferably, the expression of a combination of at least two, three, four, five, six, seven, eight, nine or ten genes is determined.

In a preferred embodiment, the expression of at least one gene selected from the group comprising MIDI, EHF, ARHGAP29, A4GALT, CYP1A1, PRKACB, GJC1, BCL2L1, FAM122B, INPP4B, BDNF, PPAP2B, ERII, SERINC2, ANXA10, CYP24A1, CAMK2D, HTR7, TBX3, TSPAN1 and KRT7 is determined. More preferably, the expression of a combination of at least two, three, four, five, six, seven, eight, nine or ten genes is determined. Most preferably, the expression of all genes MIDI, EHF, ARHGAP29, A4GALT, CYP1A1, PRKACB, GJC1, BCL2L1, FAM122B, INPP4B, BDNF, PPAP2B, ERII, SERINC2, ANXA10, CYP24A1, CAMK2D, HTR7, TBX3, TSPAN1, and KRT7 is determined.

In another preferred embodiment, the expression of at least one gene selected from the group comprising CYP24A1, EHF, KRT7, PRKACB and ANXA10 is determined. More preferably, the expression of a combination of at least two, three, four or five genes is determined. Most preferably, the expression of all genes CYP24A1, EHF, KRT7, PRKACB and ANXA10 is determined.

Furthermore, the present invention provides a method for determining the sensitivity of a patient suffering from a cancer disease to Aurora kinase inhibitor therapy, which comprises determining *in vitro* in the cancer cells taken from the patient the level of at least one protein selected from the group comprising:

| **Protein Name** | **Change in level determining resistance** | **Resistant clones** |
|---|---|---|
| Chloride intracellular channel protein 1 | Decrease (-1.4) | p53-/-: ZM clones |
| Isocitrate dehydrogenase [NAD] subunit alpha, mitochondrial | Decrease (-2.32) | p53+/+: ZM clones |
| Keratin, type II cytoskeletal 18 | Decrease (-2.14) | p53-/-: ZM clones |
| Keratin, type I cytoskeletal 19 | Decrease (-2) | p53-/-: ZM clones |
| Rab GDP dissociation inhibitor beta | Decrease (-1.7) | p53+/+: ZM clones |
| Splicing factor, arqinine/serine-rich 7 | Decrease (-2.31) | p53+/+: ZM clones |
| Platelet-activating factor acetylhydrolase IB subunit beta | Decrease (-2.16) | p53-/-: ZM clones |
| Serpin B5 | Increase (2) | p53+/+: ZM clones p53-/-: ZM clones |
| Ras GTPase-activating protein-binding protein 1 | Increase (2) | p53-/-: ZM clones |
| Ubiquitin carboxyl-terminal hydrolase isozyme L3 | Increase (1.4) | p53-/-: ZM clones |
| Phosphoserine phosphatase | Increase (2.09) | p53-/-: ZM clones |
| 78 kDa glucose-regulated protein | Decrease (-2.10) | p53-/-: ZM clones |
| Elongation factor 1-delta | Decrease (-2.16) | p53-/-: ZM clones |
| Heat shock cognate 71 kDa protein | Increase (2.2) | p53+/+: ZM clones p53-/-: ZM clones |
| Phosphoglycerate mutase 1 | Increase (2.09) | p53+/+: ZM clones |
| GTP-binding nuclear protein Ran | Increase (2) | p53+/+: ZM clones |
| Fascin | Increase (2) | p53-/-: ZM clones |
| Proteasome subunit beta type-2 | Increase (2.08) | p53+/+: ZM clones |
| Heterogeneous nuclear ribonucleoprotein H | Decrease (-5.58) | p53+/+: ZM clones |
| Phosphoserine aminotransferase | Increase (2.46) | p53-/-: ZM clones |
| Eukaryotic translation initiation factor 4H | Increase (2.28) | p53+/+: ZM clones |
| Annexin A3 | Increase (2.03) | p53+/+: CYC116 clones |
| Tropomyosin alpha-4 chain | Decrease (-4.32) | p53+/+: CYC116 clones |
| Gamma-enolase | Increase (2.43) | p53+/+: CYC116 clones |
| Splicing factor, arqinine/serine-rich 7 | Decrease (-2.81) | p53-/-: CYC116 clones |
| Serpin B5 | Increase (2.6) | p53+/+: CYC116 clones p53-/-: CYC116 clones |
| Heterogeneous nuclear ribonucleoprotein G | Decrease (-2.3) | p53+/+: CYC116 clones p53-/-: CYC116 clones |
| Heat shock protein HSP 90-beta | Increase (2.82) | p53-/-: CYC116 clones |
| dCTP pyrophosphatase 1 | Decrease (-3.81) | p53-/-: CYC116 clones |
| Inositol-3-phosphate synthase 1 | Increase (2) | p53+/+: CYC116 clones |
| Nucleophosmin | Increase (2) | p53-/-: CYC116 clones |
| Ras-related protein Rab-1 B | Increase (2.2) | p53+/+: CYC116 clones p53-/-: CYC116 clones |
| Heat shock cognate 71 kDa protein | Increase (2.05) | p53+/+: CYC116 clones |
| Eukaryotic translation initiation factor 3 subunit G | Increase (2.05) | p53-/-: CYC116 clones |
| Inosine triphosphate pyrophosphatase | Increase (2.22) | p53+/+: CYC116 clones |
| Heat shock protein HSP 90-alpha | Decrease (-2.13) | p53+/+: CYC116 clones |
| Calretinin | Increase (5) | p53+/+: CYC116 clones |
| Serine/arginine-rich splicing factor 2 | Decrease (-4.44) | p53+/+: CYC116 clones |
| Heterogeneous nuclear ribonucleoprotein L | Decrease (-2.09) | p53+/+: CYC116 clones |
| Heterogeneous nuclear ribonucleoprotein H3 | Decrease (-2.1) | p53+/+: CYC116 clones p53-/-: CYC116 clones |
| Pyruvate kinase isozymes M1/M2 | Increase (2.38) | p53+/+: CYC116 clones |
| 6-phosphofructokinase type C | Decrease (-2.11) | p53-/-: CYC116 clones |
| Voltage-dependent anion-selective channel protein 2 | Increase (2.05) | p53+/+: CYC116 clones |
| Voltage-dependent anion-selective channel protein 1 | Increase (2.36) | p53+/+: CYC116 clones |
| Serine hydroxymethyltransferase, mitochondrial | Increase (1.6) | p53+/+: CYC116 clones p53-/-: CYC116 clones |
| Phosphoserine aminotransferase | Increase (2.71) | p53-/-: CYC116 clones |
| Malate dehydrogenase, mitochondrial | Increase (2.56) | p53+/+: CYC116 clones |

Fold changes between the controls and resistant clones were calculated by REDFIN software from the mean normalized spot volumes (p-value <0.05).
The controls to which the drug resistant cancer cells are compared are usually their genetically identical drug sensitive counterparts.

The regulated proteins were identified by comparative 2-D gel electrophoresis in the pH range 4-7 and 6-11 followed by MALDI/TOF/TOF protein identification. Altogether there are 43 proteins whose expression changed about 2 fold or > 2 fold, about -2 fold or < -2 fold in the resistant cells compared to parent drug sensitive cells.

Preferably, the levels of a combination of at least two, three, four, five, six, seven, eight, nine or ten proteins is determined.

The Aurora kinase inhibitor is preferably selected from CYC116 (4-methyl-5-(2-(4-morpholinophenylamino)pyrimidin-4-yl)thiazol-2-amine), ZM447439 (N-[4-[[6-Methoxy-7-[3-(4-morpholinyl)propoxy]-4-quinazolinyl]amino]phenyl]benzamide), AZD1152 (2-[ethyl-[3-[4-[[5-[2-(3-fluoroanilino)-2-oxoethyl]-lHpyrazol3yl]amino]quinazolin7-yl]oxypropyl]amino]ethyl dihydrogen phosphate ), VX-680 (N-[4-[4-(4-methylpiperazin-l-yl)-6-[(5-methyl-1H-pyrazol-3-yl)amino]pyrimidin-2-yl]sulfanylphenyl]cyclopropanecarboxamide), MLN8054 (4-[[9-chloro-7-(2,6-dfluorophenyl)-5H-pyrimido[5 4-d][2]benzazepin-2-yl]amino]benzoic acid ), MLN8237 (4-[[9-chloro-7-(2-fluoro-6-methoxyphenyl)-5H-pyrimido[5,4-d][2]benzazepin-2-yl]amino]-2-methoxybenzoic acid), PHA-739358 (N-[5-[(2R)-2-methoxy-2-phenylacetyl]-4,6-dihydro-1H-pyrrolo[3,4-c]pyrazol-3-yl]-4-(4-methylpiperazin-1-yl)benzamide), AT-9283 (1-cyclopropyl-3-[(3Z)-3-[5-(morpholin-4-ylmethyl)benzimidazol-2-ylidene] -1,2-dihydropyrazol-4-yl]urea).

The methods suitable for the determination of the expression include immunochemical methods, immunohistochemical methods, immunocytochemical methods, immunofluorescence techniques, PCR (RT-PCR), electrophoresis, mass spectrometry, and ELISA.

The cancer diseases, for which the method of the present invention is useful, include sarcomas, colorectal, melanoma, skin, breast, thyroid, glioblastoma, lung, prostate, ovarian, cervical, uterine, head and neck, hematological, gastric, oesophageal, neural, pancreatic, and renal cancers.

Furthermore, this invention also includes Bcl-2 inhibitors, in particular those selected from the group comprising ABT-263 [(R)-4-(4-((4'-chloro-4,4-dimethyl-3,4,5,6-tetrahydro[1,1'-biphenyl]-2-yl)methyl)piperazin-1-yl)-N-((4-((4-morpholino-1-(phenylthio)butan-2-yl)amino)-3-((trifluoromethyl)sulfonyl)phenyl)sulfonyl)benzamide], AT-101 (7-(8-formyl-1,6,7-trihydroxy-3-methyl-5-propan-2-ylnaphthalen-2-yl)-2,3,8-trihydroxy-6-methyl-4-propan-2-ylnaphthalene-1-carbaldehyde), GX15-070 (2E)-2-[(5E)-5-[(3,)5-dimethyl-1H-pyrrol-2-yl)methylidene]-4-methoxypyrrol-2-ylidene]indole;methanesulfonicacid), TW-37 (N-[4-(2-tert-butylphenyl)sulfonylphenyl]-2,3,) 4-trihydroxy-5-[(2-propan-2-ylphenyl)methyl]benzamide), and sHA 14-1 (2-amino-6-bromo-4-(1-cyano-2-ethoxy-2-oxoethyl)-4H-chromene-3-carboxylate), in combination with an Aurora kinase inhibitor for use in the treatment of Aurora kinase inhibitor-resistant tumors.

We have found out that Bcl-2 inhibitors, e.g., ABT-263, surprisingly overcome the resistance of tumors to Aurora kinase inhibitors.

More particularly, the Bcl-2 inhibitors were shown to overcome the resistance in Bcl-xL overexpressing p53 wild type CYC116, which were determined both at RNA and protein level.

To validate the role of Bcl-xL overexpression in Aurora kinase (e.g., CYC116) induced resistance, we also used RNA interference method to knock down Bcl-xL expression genetically followed by Aurora kinase inhibitor treatment. In correspondence with the Bcl-2 inhibitor ability (pharmacologically) to reverse the resistance, combination of anti-Bcl-xL siRNA and Aurora kinase inhibitor restored the sensitivity (close to parent cell line) of resistant tumors towards Aurora kinase inhibitor.

ABT-263 [((R)4-(4-((4'-chloro-4,4-dimethyl-3,4,5,6-tetrahydro[1,1'-biphenyl]-2-yl)methyl)piperazin-1-yl)-N-((4-((4-morpholino-1-(phenylthio)butan-2-yl)amino)-3-((trifluoromethyl)sulfonyl)phenyl)sulfonyl)benzamide)] is a novel pan-Bcl-2 inhibitor. ABT-263 is orally available Bad-like BH3 mimetic with Ki's of <1 nM/L for Bcl-2, Bcl-xL, and Bcl-w. Bcl-2 family members particularly Bcl-2, Bcl-xL, and Bcl-w overexpression has been shown to associate with tumor cell resistance and progression. ABT-263 disrupts association of Bcl-2Bc1-xL with pro-apoptotic proteins (Bim), which results in the rapid apoptotic cell death (Tse C. et al., Cancer Research 2008; 68, 3421-3428). It was also shown to enhance the activity of chemotherapeutic agents in xenograft models.

Currently, several other Bcl-2 inhibitors are in clinical and preclinical studies. AT-101 (7-(8-formyl-1,6,7-trihydroxy-3-methyl-5-propan-2-ylnaphthalen-2-yl)-2,3,8-trihydroxy-6-methyl-4-propan-2-ylnaphthalene-1-carbaldehyde) developed by Ascenta therapeutics is an orally available potent inhibitor of Bcl-2, Bcl-xL, and Mcl-1. It is currently in phase II clinical trials being tested in solid and blood cancers (Liu G. et al., Clinical Cancer Research 2009; 15, 3172-3176). It exhibited significant anti-tumor activity in several tumor models including breast, colon, prostrate, head and neck, chronic lymphocytic leukemia, non-Hodgkin's lymphoma, and multiple myeloma. The compound was well tolerated with less severe toxicities, which include diarrhea, fatigue, nausea, and anorexia. This compound has good pharmacokinetic and pharmacological properties. Obatoclax mesylate (GX15-070) (2E)-2-[(SE)-5-[(3,)5-dimethyl-1H-pyrrol-2-yl)methylidene]-4-methoxypyrrol-2-ylidene]indole;methanesulfonicacid) developed by Gemini X is a potent inhibitor of Bcl-2, Bcl-xL, Bcl-w, A1, and Bcl-b. It is currently in phase II clinical studies being tested in solid and hematological cancers (Schimmer A.D. et al., Clinical Cancer Research 2008; 14, 8295-8301). It is available in the form of infusions to the patients. The side effects of Obatoclax include somnolence, fatigue, dizziness, euphoric mood, and gait disturbance. The plasma concentrations reached to a steady state before the end of infusion.
Several Bcl-2 inhibitors are currently under preclinical evaluation. TW-37 (N-[4-(2-tert-butylphenyl)sulfonylphenyl]-2,3,) 4-trihydroxy-5-[(2-propan-2-ylphenyl)methyl]benzamide) was first synthesized by researchers at Michigan University. It has high affinities towards Bcl-2, Bcl-xL, and Mcl-1. It has both pro-apoptotic (Mohammad R.M. et al., Clinical Cancer Research 2007; 13, 2226-2235) and antiangiogenic activities (Zeitlin B.D. et al., Cancer Research 2006; 66, 8698-8706). TW-37 was given as i.v. in mice. The side effects in mice at MTD include weight loss and scruffy fur. Preclinical sHA 14-1 (2-amino-6-bromo-4-(1-cyano-2-ethoxy-2-oxoethyl)-4H-chromene-3-carboxylate) has high affinity towards Bcl-2, Bcl-xL, and Bcl-w. It induced apoptosis effectively in Jurkat cells (Tian D. et al., Cancer Letters 2008; 8, 198-208) It was also shown to overcome drug resistance. Some of the naturally occurring Bcl-2 inhibitors include tetrocarcin A, chelerythrine chloride and antimycin. Apart from these, several pharmaceutical companies are developing their lead Bcl-2 inhibitors.
Potentially all the above described Bcl-2 inhibitors can be used in combination with Aurora kinase inhibitors to overcome the drug resistance.

Bcl-xL expression was also shown as a possible indicator of chemoresistance in multiple myeloma (Tu Y. et al., Cancer Research 1998; 58, 256-62). Hence overexpression of antiapoptotic Bcl-2 members forms a strong rationale to target by small molecule inhibitors. ABT-263 is currently in phase II clinical trial being evaluated in many solid cancers and refractory leukemia's.

The action of ABT-263 which is shown in one example of the present application to overcoming the resistance towards Aurora kinase inhibitors, which is clearly connected, inter alia, with changes in expression of the Bcl family, indicates that Bcl-2 inhibitors in general are suitable for overcoming the resistance of tumors towards Aurora kinase inhibitors. Particularly upregulation of Bcl-xL (Bcl-2 family member) in HCT116: CYC116 resistant clones were also determined at protein level by using western blot. Hence we tested ABT-263, a Bcl-2 family inhibitor on CYC116 resistant clones in an effort to overcome the drug resistance.

The names and abbreviations of the genes are shown in accordance with ENSEMBL and Affymetrix databases.

### Brief Description of Drawings

Figure 1: In comparison of resistant clones gene expression profiles in primary tumor samples (see example), the Ct values for several genes (see Table 8) were used to construct a chart to show the relative gene expression in drug sensitive versus drug resistant patient tumors.
Figure 2: Efficacy of ABT-263 on CYC116 and ZM447439 resistant clones. The Y-axis represents IC50 values (µM) of ABT-263 on parent and resistant clones. The MTT assay was performed in three independent replicates (n=3).
Figure 3: Western blot showing the upregulation of Bcl-xL in HCT116: CYC116 resistant clones in comparison to HCT116 parent cell line. Actin was used as a loading control.
Figure 4: MTT assay showing genetic (siRNA) Bcl-xL knockdown followed by CYC116 treatment, restored the sensitivity of CYC116 resistant clone towards CYC116 (n = 3).

### Examples of carrying out the Invention

### EXAMPLE 1

### Introduction

We used two cell lines (HCT116 p53+/+ and HCT116p53-/-) and two Aurora kinase inhibitors (CYC116 and ZM447439) to select resistant clones. Exposed each cell line separately to either CYC116 or ZM447439 at 1 µM concentration, after 4-5 weeks colonies appeared. Colonies were isolated and bulked up for further studies.
Preliminary characterization of resistant clones was done in relation to their resistance, cross-resistance, multidrug resistance, cell cycle profile, expression of drug transporter, and biomarker modulations. All the CYC116 and ZM447439 resistant clones displayed cross-resistance to other Aurora kinase inhibitors (Table 1), which are structurally quite distinct. Those inhibitors include AZD1152 (AstraZeneca's Aurora B specific), VX-680 (Vertex's pan-Aurora inhibitor, and MLN8054 (Millenniums Aurora A specific). This cross-resistance is primarily due to their similar mechanistic actions and the molecular basis of resistance could be common. Hence our inventions can be applied to the Aurora kinase inhibitors which are already in the clinical trials (AZD1152, VX-680, and MLN8054) and to the inhibitors being developed.

**Table 1. Cross-resistance profile of CYC116 and ZM447439 resistant clones to other synthetic Aurora kinase inhibitors**

| **Cell line or Resistant Clone** | **AZD1152** | **VX-680** | **MLN8054** |
|---|---|---|---|
| **HCT116 p53+/+ parent** | 0.01 | 0.03 | 0.19 |
| **HCT116 p53-/- parent** | >50 | 0.1 | 0.17 |
| **CYC116 (p53+/+ resistant clones)** | | | |
| R1.1 | 17(1700) | 1.9 (63) | 31 (163) |
| R1.2 | 18(1800) | 2.0 (67) | 15 (79) |
| R1.3 | 11 (1100) | 1.0 (33) | 16 (84) |
| **CYC116 (p53-/- resistant clones)** | | | |
| R2.1 | >50 | 4.0(40) | 30(176) |
| R2.2 | >50 | 2.0 (20) | 3 (18) |
| R2.3 | >50 | 2.4 (24) | 18(106) |
| **ZM447439 (p53+/+ resistant clones)** | | | |
| R3.1 | 36 (3600) | 2.6 (87) | 2.0(10) |
| R3.2 | 8 (800) | 0.7 (23) | 2.0(10) |
| R3.3 | 0.07 (7) | 0.09 (3) | 0.4(2) |
| **ZM447439 (p53-/- resistant clones)** | | | |
| R4.1 | >50 | 0.8 (8) | 22 (129) |
| R4.2 | >50 | 1.5 (15) | 18.6(109) |
| R4.3 | >50 | 3.0(13) | 39 (229) |

All the values in the above table represent mean IC50s in µM calculated from three independent experiments, each done in 2 technical replicates. The SD values for the above data are in the range ±0.0004 - ±11. The values in parentheses are fold increase calculated by dividing mean IC50 value of respective clones by the IC50 values of parent p53+/+ or p53-/- cells. AZD1152 was unable to reach IC50 value on p53-/- back ground cells even at the highest concentration tested.

Methods used to identify potential resistance mechanisms include analysis of drug transporters expression, Aurora kinases expression, mutations of target, and microarray based differential gene expression. The gene expression signatures determined in CYC116 resistant clones were compared to various CYC116 sensitive and resistant primary tumor biopsies. Comparative genomic hybridization was performed for all the resistant clones to determine structural and numerical changes of genes. Finally differential protein expression studies were performed by 2DE and mass spectrometry.

### Examples of specific genes that are highly up-regulated (>2 fold change) or down-regulated (<2 fold change) and their biological roles are shown below:

Cytochrome P450, family 1, subfamily A, polypeptide 1 (CYP1A1) was found to highly overexpress in all CYC116 resistant clones. CYP1A1 is involved in the metabolism of polycyclic aromatic hydrocarbons (PAH). In tobacco smokers CYP1A1 transforms PAH into procarcinogens. CYP1A1 expression was reported in pulmonary cancers and also altered expression in many lung tumors (McLemore T.L. et al., Journal of the National Cancer Institute 1990; 82, 1333-39). When HCT116 and HCT116 p53-/- treated with CYC116 for 48 h, up-regulation of CYP1A1 was not detected. However all the CYC116 resistant clones, displayed high levels of CYP1A1. Hence CYP1A1 is highly reliable marker in predicting CYC116 response and based on its function one could conclude that CYP1A1 inhibition could be used to increase metabolic stability and decrease drug resistance to CYC116.

Runt-related transcription factor 2 (RUNX2) is another gene that is up-regulated in HCT116: CYC116 clones. RUNX2 is transcription factor involved in osteoblast differentiation and also has a key role in carcinogenesis in many cancer types. It was shown to overexpress in metastasized breast and pancreatic cancers particularly to bone. It was also implicated in survival and metastasis promotion. It was found to overexpress in highly metastatic prostate cancer and helped in colony formation. Induced expression of RUNX2 in 22Rv1 prostate cancer cell line conferred resistance to anticancer agents (Chua C.W. et al., Clinical Cancer Research 2009; 15, 4322-35).

v-Akt, murine thymoma viral oncogene homolog 3 (protein kinase B, gamma) (AKT3) is up-regulated in HCT116: CYC116 clones. De-regulated AKT isoforms inactivates some of the important pro-apoptotic genes (BAD and procaspase-9) and induces tumor cell survival. It was also shown to activate MDM2 activation and subsequent p53 down-regulation. Knock-down of AKT induced apoptosis in many cancer cell lines (Koseoglu S. et al., Cancer Biology & Therapy 2007; 6, 755-62). Hence AKT will serve as reliable biomarkers while assessing CYC116 response. Recently its role in resistance towards B-RAF targeted melanoma cells was described (Shao Y. et al., Cancer Research 2010; 70, 6670-81).

Keratin 7 (KRT7) are also up-regulated in HCT116: CYC116 clones. Cytokeratins are structural proteins, which form a frame work for integrity, signal transduction, and differentiation. Cytokeratins were shown to influence cancer cell survival in response to chemotherapy. Expression of cytokeratins conferred multidrug resistance to several anticancer agents. Increased expression of cytokeratins may affect drug distribution, sparing nuclear targets like oncogenic Aurora kinases (Bauman P.A. et al., Proceedings of the National Academy of Sciences of the United States of America 1994; 91, 5311-14).

Cytochrome P450, family 24, subfamily A, polypeptide 1 (CYP24A1) is highly down-regulated in both HCT116 and HCT116 p53-/- CYC116 resistant clones. It is involved in the degradation of active vitamin-D. CYP24A1 was shown to overexpression in many cancers and it is associated with poor prognosis. Active vitamin-D has anticancer activity in lung adenocarcinoma cells. CYP24A1 mRNA is highly expressed in poorly differentiated cancers. A549 cell line was more resistant to vitamin-D because of high CYP24A1 expression (Chen G. et al., Clinical Cancer Research 2011; 17, 817-26). However the down-regulation mechanism of CYP24A1 and its effects in CYC116 resistant clones is unknown, but may be associated with slower cycling of resistant cells and thus increased response to Aurora kinase inhibition.

Ets homologous factor is highly up-regulated in HCT116 p53-/-: CYC116 resistant clones. EHF has conserved DNA binding domain and its aberrant expression was reported in many cancers. In response to doxorubicin induced stress, EHF expression lead to decreased senescence and doxorubicin resistance in prostate cancer cell line. Knock-down of EHF inhibited cell growth and induced senescence (Park C. et al., Molecular Cancer Therapeutics 2006; 5, 3191-96). In the same study telomerase was shown to up-regulate in the presence on EHF.

Pre-B-cell leukemia homeobox (PBX1), which is up-regulated in HCT116 p53-/-: CYC116 clones. It is a transcription factor involved in the regulation of cell survival and differentiation. PBX1 positively regulates valosin-containing protein, which is involved in cancer cell growth. Knock-down of PBX1 gene reduced VCP expression. Decreased expression of PBX1 significantly reduced viability after TNFα treatment (Qiu Y. et al., Epithelial and Mesenchymal Cell Biology 2007; 170, 152-9). Thus PX1 and VCP expression is important for cell survival under cytokine stress

Midline 1 (Opitz/BBB syndrome) (MIDI) is highly downregulated in HCT116 p53-/-: CYC116 resistant clones. aCGH studies revealed deletion of MID1, which corresponded to high down-regulation of MID1. Mutations of MID1 causes Opitz/BBB syndrome, characterized by midline abnormalities (Perry J. et al., Genomics 1999; 62, 385-94). It has been shown associate with microtubules throughout the cell cycle and to midbody during cytokinesis. Aurora kinases also have similar localization during mitosis. The down-regulation mechanism in CYC116 resistant clones is unknown, but may be associated with slower cycling of resistant cells and thus decreased response to Aurora kinase inhibition. Nevertheless MID1 can be used a robust marker to predict CYC116 response.

ABCF1, a member of the ATP-binding cassette transporter family is up-regulated in HCT116: ZM447439 resistant clones. These proteins are well characterized transporters of many anticancer drugs. Some of the drug transporters were shown to overexpress in resistance tumors. For example ABCB1 (PgP) was shown to transport many anticancer agents including taxol (Parekh H. et al., Biochemical Pharmacology 1997; 4, 461-70), imatinib (Illmer T. et al., Leukemia 2004; 18, 401-8), and anthracyclines (Hu X.F. et al., British Journal of Cancer 1995; 71, 931-36).

Annexin 10 (ANXA10) is significantly down-regulated in HCT116: ZM447439 resistant clones. Annexins are membrane proteins involved in the regulation of the signal transduction and cell growth. Decreased expression was reported in gastric cancer tissues compared to normal cells. Transfection of ANXA10 gene in these cell lines inhibited cell growth with augmented apoptosis (Kim J.K. et al., Oncology Reports 2010; 24, 607-12).

Brian-derived neurotrophic factor (BDNF) is down-regulated in HCT116: ZM447439 resistance clones. BDNF in co-ordination with TrkB tyrosine kinase is mainly involved in the survival of neurons of the brain. Increased expression of BDNF is associated with poor prognosis particularly in neuroblastoma. BDNF was shown to mediate paclitaxel resistance in neuroblastoma by down-regulation pro-apoptotic Bim (Li Z. et al., Cell Death and Differentiation 2006; 14, 318-26).

Caveolin-1 (CAV1) is significantly down-regulated both in HCT116 and HCT116 p53-/-: ZM447439 resistant clones. Caveolae are membrane proteins and have been implicated in several signaling pathways. CAV1 role as tumor suppressor has been described previously. Its expression was shown to be down-regulated in some liposarcomas, fibrosarcomas, and angiosarcomas. Forced expression of CAV1 in HT-1080 fibrosarcoma cell line inhibited colony formation (Wiechen K. et al., The American Journal of Pathology 2001; 158, 833-39). This work clearly provides evidence of CAV1 as tumor suppressor and its downregulation contributes drug resistance.

Up-regulation of Bcl-xL (BCL2L1) was found in both HCT116 and HCT116 p53-/-: CYC116 resistant clones. Bcl-xL is a potent inhibitor of apoptotic cell death. Bcl-xL inhibits pro-apoptotic Bax translocation into mitochondria, cytochrome c release, and caspase-3 cleavage (Ackler S. et al., Cancer Chemotherapy and Pharmacology 2010; 66, 869-80). Up-regulation of Bcl-xL was correlated to decreased response to melphalan and prednisone or vincristine, Adriamycin, and dexamethasone in multiple myeloma patients. Particularly Bcl-xL expression is frequent in biopsies taken from the patients at relapse (Tse C. et al., Cancer Research 2008; 68, 3421-3428).

### Determination of global gene expression by Human Gene 1.0 ST Array (Affymetrix)

The fold changes of specific gene by Human Gene 1.0 ST Array can be conveniently performed from any cancer cell line, given the conditions that we have sufficient quantity and quality of RNA. RNA was isolated in three biological replicates from all the healthily dividing resistant clones and controls. 10 x 10⁶ cells were used to isolate the RNA. The cells were lysed using 1 ml of TRI reagent. 200 µl of chloroform was added to TRI reagent and allowed to incubate for 10 minutes at room temperature, followed by centrifugation for 15 min at 12,000 g, 4°C. The solution separates into three phases. The upper RNA portion is collected carefully, followed by RNA precipitation using 500 µl isopropanol. Subsequent centrifugation and washing with 75% of ethanol yielded RNA pellet. DEPC water was added according to size of the RNA pellet.
For preparation of labeled sense target 300 ng of RNA as a starting material was used. The samples were processed and hybridized to Affymetrix chip following manufacturer's instructions. RNA was isolated from cell lines using TRI reagent. 300 ng of RNA was used for preparation of biotinylated sense-strand DNA targets according to Affymetrix protocol. The fragmented and labeled sample was hybridized to Affymetrix Human Gene 1.0 ST array. Expression profiles were examined from three independent biological replicates. All statistical analyses of expression arrays were carried out using either an assortment of R system software (http://www.R-project.org, version 2.11.0) packages including those of Bioconductor (version 2.7) by Gentleman et al. (Gentleman R.C. et al., Genome Biology 2004; 5, R80) or original R code. We used the affyQCReport Bioconductor R package to generate a QC report for all chips. Chips that did not pass this filter were not included in this study. Raw feature data from the expression chips were normalized in batch using robust multi-array average (RMA) method by Irizarry et al. (Irizarry R.A. et al., Biostatistics 2003; 4, 249-64) implemented in R package affy. Based on the RMA log₂ single-intensity expression data, we used Limma moderate T-tests (Bioconductor package limma) (Smyth G.K. et al., Springer 2005; 397-420) to identify differentially expressed genes. The p.adjust function from stats R package was used to estimate the FDR using the Benjamini-Hochberg (BH) method (Benjamini Y. et al., Journal of Royal Statistical Society Series B 1995; 57, 289-300).

### Comparison of gene expression profiles in primary tumor samples

From each group of resistant clones, top 100 gene hits were listed according to decreasing p-value. Common genes between the relevant groups, genes which were highly upregulated or downregulated, and some based on biological relevance were selected for qRT-PCR validation studies (totally 42 genes). Out of 42 genes from primary resistant cells, 12 genes were selected (qRT-PCR) for comparison and validation in primary tumor samples. Previously we tested the sensitivity of CYC116 on various primary tumors using 96-h MTT assay. 13 CYC116 sensitive primary tumors and 14 CYC116 resistant tumors were selected for selected gene expression studies using qRT-PCR. Any primary tumor samples which are well cryopreserved are suitable to isolate high quality RNA. The RNA was isolated from primary tumor samples as described previously for resistant cell lines. 4.5 µg of RNA was used for preparation of cDNA in a total volume of 45 µl reaction mix. Mixture of 4.5 µl RNA, 0.45 µg hexamer is completed by water to 19.5 µl and incubated in a thermocycler at 70° C for 5 minutes. After incubation the samples were placed on ice for 1 minute. Master mix prepared from 9 µl 5x RT buffer, 4.5 µl 10 mM dNTP, and 1.125 µl (30 U) RNAsin was added to each sample. Finally 150 U of reverse transcriptase was added, mixed and incubated at room temperature for 10 minutes. Following this the samples were incubated in a gradient thermocycler at 42° C for 60 minutes and 70° C for 10 minutes. After incubation time, the samples were stored at -20° C.

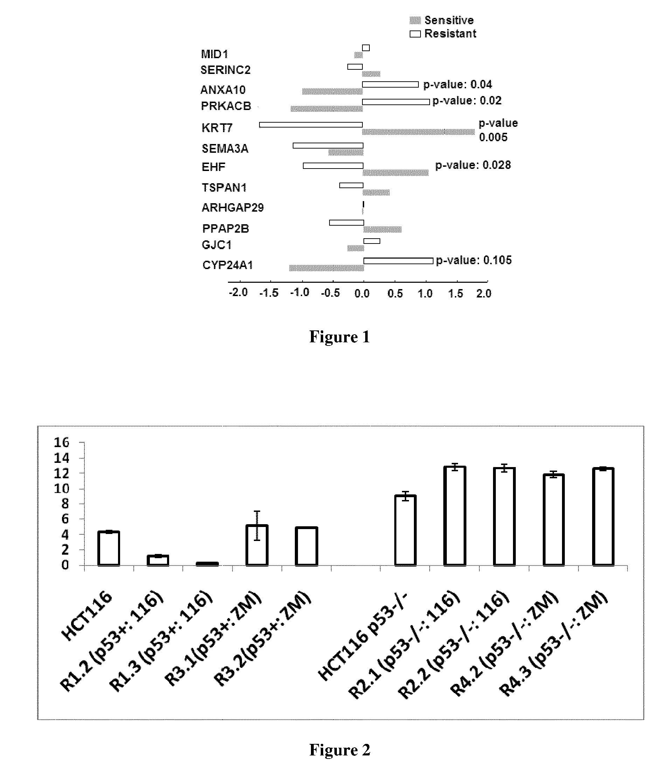

100 ng of cDNA was used to perform RT-PCR in a total reaction volume of 25 µl. The RT-PCR we performed was based on the SYBR green binding capability to accumulated PCR product (target gene cDNA). Given the conditions that we have good cDNA quality and well designed highly specific primers, SYBR green can work extremely well. Master mix was prepared from 12.8 µl DEPC water, 2.5 µl 10 x PCR buffer, 3 µl of Mg 2+, 2 µl (0.005 mM) of forward and reverse primer each, 0.5 µl 10 mM dNTP, 1 µl (1:1000) SYBR green, and 0.2 µl (1 U) Taq polymerase. 24 µl of master mix was distributed to the tubes. The tubes were loaded into the carousel, performed automatic calibration by sensing the fluorescence and started the relevant program. The Ct (Cycle threshold) values obtained for each gene in a particular sample were normalized by subtracting with the Ct values of GAPDH housekeeping gene. To calculate relative gene expression of resistant samples a statistical method was applied. First the mean was calculated (value A) from the normalized Ct values of a gene from all the sensitive and resistant samples. Then normalized Ct value of each gene from each sample was subtracted from value A. The obtained value is designated as value B for convenience. Finally the mean was calculated from the obtained values separately for sensitive sample and resistant sample groups. These values were plotted in a chart to show relative gene expression differences between the sensitive and resistant samples (Fig. 1).

The proposed gene primers were designed by using freely accessible internet server called Primer3. The proposed primers for selected genes and thermal schemes were presented in Table 2. During the optimization process the specificity of gene primers were tested and optimum melting temperature was chosen. Optimization process for all the genes were performed successfully with the proposed primers. Finally the sizes of the amplified products were verified by Agilent bioanalyzer using the DNA chips.

**Table 2. Proposed primers sequences and thermal profiles for selected genes**

| **Gene** | **Forward primer** | **Reverse primer** | **Thermal profile** | **Product size** |
|---|---|---|---|---|
| CYP24A1 | CTGGGATCCAAGGCATTCTA | ATGGTGCTGACACAGGTGAA | 95°C/15sec-62°C/ 15sec | 63 bp |
| GJC1 | ATGGTGTTACAGGCCTTTGC | GAGTCTCGAATGGTCCCAAA | 95°C/15sec -62°C/15sec | 76 bp |
| PPAP2B | AAATGACGCTGTGCTCTGTG | ACCGCGACTTCTTCAGGTAA | 95°C/15sec -62°C/15sec | 98 bp |
| ARHGAP29 | CATGGCAGCTGAATCTTTGA | AGCCAGATGACAGGAGCCTA | 95°C/15sec-62°C/15sec | 78 bp |
| TSPAN1 | CCTTTCTGCTCCAGACTTGG | AAGTCAGGCATCGCCTAAAA | 95°C/15sec -62°C/15sec | 60 bp |
| EHF | AGGTGATGCATCCTCCTCAC | AATGTTCACCTCCCTTGACG | 95° C/ 15sec -62°C/15sec | 59 bp |
| SEMA3A | TGCCAAGGCTGAAATTATCC | GCCAAGCCATTGAAAGTGAT | 95° C/ 15sec -62°C/15sec | 70 bp |
| KRT7 | GATGCTGCCTACATGAGCAA | TGAGGGTCCTGAGGAAGTTG | 95°C/15sec -62°C/15sec | 82 bp |
| PRKACB | GAGACCGTCCTTGTTGAAGC | ACGGGATGATGGCAATAAAG | 95° C/15sec -60°C/15sec | 78 bp |
| ANXA10 | GTCCTATGGGAAGCCTGTCA | GCTCTTGTTGCACAGGATCA | 95°C/15sec -60°C/15sec | 75 bp |
| SERINC2 | CGTGTGGGTGAAGATCTGTG | CAGGGTCCACAGGTAGAGGA | 95°C/15sec -66°C/15sec | 58 bp |
| MID1 | ACCCAACATCAAGCAGAACC | GGCCTTGACCATGAAGATGT | 95° C/15sec -64°C/15sec | 76 bp |

### Comparative Genomic Hybridization

aCGH analysis can be effectively used to determine the structural and numerical changes of chromosomal genes. The method can be conveniently performed from any type of cells having high quality DNA. DNA was extracted from one million cells using DNeasy Blood &Tissue kit (QIAGEN). High quality DNA from any cancer cell line and primary tumor sample is necessary for this study. Extracted genomic DNA was processed exactly according to manufacturer's protocol (Affymetrix, Santa Clara, CA). 100 ng of DNA was amplified by whole genome amplification. After product purification with magnetic beads, DNA was quantified, fragmented, labeled and hybridized to Cytogenetics Whole-Genome 2.7M array. Arrays were washed, stained and scanned. We used software Partek Genomics Suite to analyze CGH arrays (Grayson B.L. et al., BioData Mining 2011; 4, 5-11). We identified regions of significant copy number change in drug resistant and control drug sensitive cell line samples and created gene lists.

### Proteomic studies

Proteins are the ultimate biological molecules which execute their functions by interacting with other partners or through enzymatic activity. Differential proteins expression is another aspect which can be used to achieve high quality results. Proteomic methods based on two-dimensional electrophoresis was preferable technology of choice to study differential protein expression. To identify the differentially expressed proteins, spots from gels are subjected to mass-spectrometric identification. Protein extracts can be continuously prepared from any intact biological material.

### Preparation of lysates:

Resistant clones and controls were grown to nearly confluency by initially seeding 3 x 10⁶ cells in Petri dishes. The monolayer was washed three times with ice cold PBS. Then 500 µl of lysis buffer (7 M urea, 2 M thiourea, 3% w/v CHAPS, 2% v/v Nonidet 40, 5 mM TCEP, protease and phosphatase inhibitor cocktails) was added on top of the monolayer and left at room temperature for 30 minutes to optimize the protein extraction. The lysates were centrifuged at 20000 g for 1 hour at 4° C and the cleared supernatants were stored at -80° C.

### Two-dimensional electrophoresis:

Protean IEF Cell and Protean II xi cell were used to carry out 1^{st} and 2^{nd} dimensions respectively. Polyacrylamide strips with an IPG of 4-7 and 6-11 were used in IEF separation and 100 µg of proteins for pH range 4-7 and 70 µg of protein for pH range 6-11 were loaded into IPG strips. For the 4-7 pH range, 110 µl of the lysates were diluted in 230 µl of rehydration buffer (7 M urea, 2 M thiourea, 4 % CHAPS, 200 mM DeStreak reagent, 2 % IPG buffer pH 4-7, protease and phosphatase inhibitor cocktails, trace of bromophenol blue). The proteins were loaded into IPG strip 4-7 using overnight in-gel rehydration at 50 V. IEF was performed as follows: 200 V for 10 h, 600 V for 30 min, 1000 V for 30 min, and 5000 V for the time period necessary to reach 50 000 Vh in total. After this, IPG strips were equilibrated in 50 mM Tris-HCl pH 6.8, 6 M urea, 30% glycerol, 4 % SDS, and 100 mM DeStreak reagent for 25 min. For pH range 6-11, IPG strips were passively rehydrated overnight without sample in 340 µl of rehydration buffer (7 M urea, 2 M thiourea, 4% CHAPS, 30 mM DTT, 0.5% IPG buffer pH 6-11, protease and phosphatase inhibitor cocktails, trace of bromophenol blue). After 15 h, lysates were diluted to 150 µl by lysis buffer with 65 mM DTT and 0.5% IPG buffer. After 15 min, 30 mM iodoacetamide was used for alkylation of free thiol groups, followed by trace of bromophenol addition and finally cup-loading was applied. IEF was performed at 150 V for 12 h, 1000 V for 1 h, 8000 V for 3 h, and 8000 V until 20 000 Vh was reached in total. The IPG strips 6-11 were equilibrated in 50 mM Tris-HCl pH 6.8, 6 M urea, 30% glycerol, 8% SDS for 20 min.

For MS identification 500 µg (pH 4-7) and 130 µg (pH 6-11) of protein were loaded into IPG strips. Proteins were reduced with 30 mM DTT and focused as described above. IPG strips were equilibrated for 15 min in 50 mM Tris-HCl pH 6.8, 6 M urea, 30% glycerol, 4% SDS, and 1% DTT. The alkylation of the free thiol groups was performed using the solution where 1% DTT is replaced with 4% iodoacetamide and a trace of bromophenol blue is present.

After equilibration, the IPG strips were placed on 10% SDS-PAGE gels and electrophoresis was carried out at 40 mA. Analytical gels were stained with SYPRO Ruby protein gel stain. Protein spots on preparative gels were visualized by reverse staining using a zinc salt (Hardy 2004). Analytical gels were scanned and digitized at 500 DPI resolution using a Pharos FX scanner. 2D gel images were then evaluated using REDFIN software. The automatically generated spot detection and matching were manually checked and regulated protein spots were searched based on the fold-change which is calculated from the mean normalized volumes between the groups of a particular comparison. Differential spots having fold-change > 1.2 and *p*-value < 0.05 (ANOVA) were considered as significant. Four biological replicates of each sample were analyzed in 2-DE. Cells were grown in different Petri dishes and all the following manipulations were performed independently.

### Enzymatic in-gel digestion:

Excised protein spots from zinc stained preparative gels were cut into small pieces. Gel pieces were incubated for minutes in 200 µl of 50 mM Tris-HCl pH 8.3, 20 mM glycine, and 30 % acetonitrile to remove zinc salt. After complete destaining, gels were washed twice with 50 mM Tris-HCl pH 8.3. Gels were then washed with water, shrunk by dehydration in MeCN and this step was repeated twice. Finally supernatant was removed and the gels were partly dried using SpeedVac concentrator. Rehydration was performed in cleavage buffer (25mM 4-ethylmorpholine acetate, 5% MeCN, 3.3 ng/µl trypsin) at 37°C overnight. The digestion was stopped using 5% trifluoroacetic acid in MeCN and the resulting peptide mixture was desalted using a GELoader microcolumn packed with a Poros Oligo R3 material. Purified and concentrated peptides were eluted from the microcolumn in several droplets directly onto MALDI plate using 1 µl of α-cyano-4-hydroxycinnamic acid matrix solution (5 mg/mL in 50% MeCN, 0.1% trifluoroacetic acid).

### Protein identification by MALDI MS:

MALDI mass spectra were measured on an Ultraflex III MALDI-TOF/TOF instrument (Bruker Daltonics) equipped with a smartbeam solid state laser and LIFT technology for MS/MS analysis. PMF spectra were acquired in the mass range of 700-4 000 Da and calibrated internally using the monoisotopic [M + H]⁺ ions of trypsin autoproteolytic fragments (842.5 and 2 211.1 Da). For PMF database searching, peak lists in XML data format were created using flexAnalysis 3.0 program with SNAP peak detection algorithm. No smoothing was applied and maximal number of assigned peaks was set to 50. After peak labeling, all known contaminant signals were removed. The peak lists were searched using in-house MASCOT search engine against Swiss-Prot 2010_09 database subset of human proteins with the following search settings: peptide tolerance of 30 ppm, missed cleavage site value set to one, variable carbamidomethylation of cysteine, oxidation of methionine and protein N-terminal acetylation. No restrictions on protein molecular weight and pI value were applied. Proteins with Mascot score over the threshold 56 were considered as identified under the fixed parameters. If the score was lower or only slightly higher than the threshold value, the identity of protein candidate was confirmed by MS/MS analysis. In addition to the above-mentioned MASCOT settings, fragment mass tolerance of 0.6 Da and instrument type MALDI-TOF/TOF was applied for MS/MS spectra searching

### Results

### Global gene expression analysis

Altogether we used two cell lines (HCT116 p53+/+ and HCT116p53-/-) and two Aurora kinase inhibitors (CYC116 and ZM447439) to select resistant clones. Exposed each cell line separately to either CYC116 or ZM447439 at 1 µM concentration, after 4-5 weeks colonies appeared. Colonies were isolated and bulked up for further studies. The resistant clones in each group were designated as follows. [1] HCT116: CYC116 (R1.1, R1.2, R1.3) [2] HCT116 p53-/-: CYC116 (R2.1, R2.2, R2.3) [3] HCT116: ZM447439 (R3.1, R3.2, R3.3) [4] HCT116 p53-/-: ZM447439 (R4.1, R4.2, R4.3).
Affymetrix based gene expression (Human Gene 1.0 ST Array) analysis revealed differential expression of many genes in the clones from each group compared to controls. Some genes differential expression is statistically significant. 885, 1085, 224, and 212 number of gene sets were differentially expressed (ANOVA pH0.001) in HCT116: CYC116 clones, HCT116 p53-/-: CYC116 clones, HCT116: ZM447439 clones, and HCT116 p53-/-: ZM447439 clones respectively. Only the top 100 are shown for each case in Tables 3 to 6. However some genes from all the three clones in each group were commonly up-regulated and some genes were commonly down-regulated. On the other hand differential expression of some genes was not common to all three clones suggesting gene expression variability in each group. Dendrogram revealed clustering of the clones together from each group. This confirms that the drug resistant gene expression signature is unique to specific Aurora kinase inhibitor, CYC116 or ZM447439 in our case, but there are also genes reflecting resistance to Aurora kinase inhibitors in general regardless p53 status or gene signatures specific for wild-type or mutant cells.
The top 100 genes with very high statistical significance were listed out. In HCT116: CYC116 group the most highly up-regulated genes within the top 100 genes include LCN2 (Average fold change: 6.6), TSPAN8 (6.55 fold), SERINC2 (5 fold), followed by HOXB6 (3.9), FXYD3 (3.7), ITGB7 (3.5), KRT13 (3.4), KLK10 (3.4 fold), SGK1 (3.34 fold), RUNX2 (3.33 fold), TBX3 (3.3 fold), TNFAIP3 (3.22 fold), CALB1 (3.2 fold), APOBEC3C (3.1 fold), AKT3 (3 fold), and PTPN22 (3 fold). The most highly down-regulated genes include CYP24A1 (-32 fold), PRKACB (-9 fold), ARHGAP29 (-4.7 fold), KLRK1 (-4.1 fold), followed by PALLD (-3.9 fold), ENC1 (-3.8), TSPAN5 (-2.8), and GJC1 (-2.7 fold). Some genes responsible for drug metabolism were also found among differentially expressed genes, which include CYP4F12 (2 fold), CYP1A1 (2.6 fold), CYP4F3 (2.2 fold), and CYP2C18 (1.2 fold). In HCT116p53-/-:CYC116 the highly up-regulated genes include EHF (8.4 fold), and CYP1A1 (8 fold), followed by PBX1 (3.9), SAMD12 (3 fold), SLC16A6 (3 fold), FSTL4 (2.8 fold), PION (2.7 fold), SYTL2 (2.67 fold), APOBEC3H (2.6 fold), and A4GALT (2.3 fold). The highly down-regulated genes include CYP24A1 (-30 fold), MIDI (-18 fold), PRF1 (-6.2 fold), ZNF22 (-4.77 fold), GJC1 (-4.7 fold), ARHGAP29 (-4.3 fold), PON3 (-4.3), TRIML2 (-3.4 fold), CDK6 (-3.1 fold), and PRKACB (-3 fold). The drug metabolism responsible genes include CYP4F11 (-2.6), CYP1B1 (4.2 fold), CYP4F12 (2 fold), and CYP4F3 (1.9 fold). Some common genes between these groups can be noticed.
In HCT116: ZM447439 group highly up-regulated genes were TUSC3 (4.6 fold), ODZ3 (4 fold), ABCF1 (3.5 fold), FAM27C (3.4 fold), CSMD3 (3.4 fold), TSPAN1 (2.6 fold), and AKT3 (2.3 fold). Some uncharacterized genes were changed more than threefold, hence annotations are not described. The highly down-regulated genes include ARMC4 (-6 fold), PALLD (-4.2) fold), MMP7 (-4.5) followed by MKX (-3.3 fold), ANXA10 (-3), MNS1 (-2.8 fold), ENC1 (-2.6 fold), BDNF (-2.5 fold), and CAV1 (-2.4 fold). In HCT116 p53-/-: ZM447439 up-regulated genes were SPARC (7 fold), EPB41L4A (5.4 fold), CD33 (3 fold) followed by LRP1B (2.9 fold), FAM198B (2.9 fold), KIRREL2 (2.8 fold), and SLC7A8 (2.6). The most highly down-regulated genes include CYP24A1 (-55 fold), MAL2 (-48 fold), SLC27A2 (-9.4 fold), LMNA1 (-9 fold), SQLE (-6 fold), followed by CAV1 (-4.3 fold), CASK (-4 fold), SYTL5 (-3.4 fold), and PDE4B (-3 fold). Eight genes are common for CYC116 clones and ZM44739 clones. Eight common genes were differentially expressed in all the groups with significant p-value <0.01, which includes ARHGAP29, HTR7, TSPAN1, ANXA10, FAM122B, ERI1, TFPI, and AP3S1.

For the differentially expressed genes the corresponding cytogenetic changes were also presented.

**Table 3. Top 100 differentially expressed genes (Cumulative p- value <0.001) and corresponding copy number changes in HCT116: CYC116 group. Chr. - Chromosome, FC - Fold change, Amp. - Amplification, Del. - Deletion, Nd - No description, fg - Family gene. For some genes, identity number is presented more than once as respective Affymetrix probe binds to one more than one location of the genome having same recognition sequence. The same Gene IDs represented more than once, have unique ENSEMBL IDs.**

| **Gene ID** | **Gene Symbol** | **Chr.** | **R1.1 logFC** | **R1.2 logFC** | **R1.3 logFC** | **logFC Mean** | **R1.1 Copy No.** | **R1.2 Copy No.** | **R1.3 Copy No.** |
|---|---|---|---|---|---|---|---|---|---|
| 8067140 | CYP24A1 | 20 | -6.68 | -3 | -6.17 | -4.99 | | | |
| 8047738 | NRP2 | 2 | 4.04 | 0.82 | 0.89 | 1.435 | | | |
| 8047763 | Nd | 2 | 4.03 | 0.45 | 1.25 | 1.309 | | | |
| 7964927 | TSPAN8 | 12 | 4.64 | 4.48 | 0.96 | 2.711 | | | |
| 7944931 | SLC37A2 | 11 | 3.79 | 1.09 | 0.66 | 1.396 | Amp. | Amp. | |
| 8016094 | GJC1 | 17 | -3.63 | -0.44 | -1.8 | -1.42 | | | |
| 8152617 | HAS2 | 8 | -0.42 | 4.5 | 1.68 | 1.476 | | | |
| 7961891 | BHLHE41 | 12 | 2.71 | -0.01 | 0.06 | 0.096 | | | Amp. |
| 7963614 | ITGB7 | 12 | 3.93 | 1.06 | 1.4 | 1.802 | | | |
| 8101828 | TSPAN5 | 4 | -4.39 | -0.78 | -1 | -1.51 | | | |
| 8150529 | DKK4 | 8 | -0.05 | -0.07 | 3.56 | 0.233 | | | |
| 8070574 | TFF2 | 21 | 2.02 | -0.25 | 0.14 | 0.411 | | Amp. | Amp. |
| 7935553 | LOXL4 | 10 | 3.21 | 0.04 | 0.77 | 0.447 | | | |
| 7943892 | NCAM1 | 11 | 2.87 | -0.1 | 2.94 | 0.944 | Amp. | Amp. | |
| 8038670 | KLK5 | 19 | 4.23 | 0.37 | 1.17 | 1.227 | | Amp. | |
| 7955613 | KRT7 | 12 | 3.71 | -0.22 | 1.29 | 1.018 | | | |
| 8158167 | LCN2 | 9 | 5.3 | 1.71 | 2.22 | 2.723 | | Amp. | |
| 8122265 | TNFAIP3 | 6 | 2.36 | 0.65 | 3.11 | 1.686 | | | |
| 8015323 | KRT13 | 17 | 5.5 | 0.72 | 1.37 | 1.755 | | Amp. | |
| 8020740 | DSG4 | 18 | 2.69 | 0.23 | -0.15 | 0.455 | | | |
| 8123936 | NEDD9 | 6 | 2.47 | 0.03 | 0.27 | 0.262 | | | Del. |
| 8173261 | ZC4H2 | X | 0.3 | -0.05 | -1.82 | -0.29 | | | |
| 8152606 | SNTB1 | 8 | 0.12 | 3.06 | 1.84 | 0.872 | | | |
| 8016994 | RNF43 | 17 | -2.98 | 0.58 | 0.09 | -0.54 | | | |
| 8168749 | SRPX2 | X | 2.71 | 0.28 | 0.78 | 0.84 | | | |
| 8112615 | ENC1 | 5 | -2.39 | -1.49 | -2.01 | -1.93 | | | |
| 7916493 | PPAP2B | 1 | 1.57 | 0.03 | 1.53 | 0.433 | | | |
| 8081548 | PVRL3 | 3 | -3.43 | 0.18 | -1.01 | -0.85 | | | |
| 8090180 | MUC13 | 3 | 1.12 | 3.14 | 0.16 | 0.818 | | Amp. | |
| 8135763 | WNT16 | 7 | -2.96 | 0.23 | -1.1 | -0.91 | Amp. | Amp. | |
| 8138566 | IGF2BP3 | 7 | -3.22 | 0.26 | 0.31 | -0.64 | Amp. | Amp. | |
| 8068633 | B3GALT5 | 21 | 2.21 | -0.16 | 0.27 | 0.454 | | | Amp. |
| 8140955 | CDK6 | 7 | -0.99 | 0.64 | 1.49 | 0.98 | Amp. | | |
| 8176174 | MPP1 | X | -1.87 | -0.06 | 0.06 | -0.19 | | | |
| 8026468 | CYP4F12 | 19 | 2.49 | 0.62 | 0.85 | 1.095 | | | |
| 8174598 | IL13RA2 | X | 3.4 | 0.58 | 0.35 | 0.881 | | | |
| 8129677 | SGK1 | 6 | 2.27 | 1.61 | 1.44 | 1.739 | | | |
| 8120043 | RUNX2 | 6 | 2.58 | 2.09 | 0.96 | 1.733 | | | |
| 8038725 | KLK10 | 19 | 3.93 | 0.78 | 1.73 | 1.746 | | Amp. | |
| 8096116 | AGPAT9 | 4 | 2.68 | 1.14 | -0.58 | 1.211 | | | |
| 8148548 | PSCA | 8 | 2.34 | -0.04 | 0.47 | 0.339 | | Amp. | |
| 8161964 | FRMD3 | 9 | 3.14 | 0.39 | 0.32 | 0.734 | | | |
| 7970954 | DCLK1 | 13 | -0.44 | 2.21 | 3.21 | 1.463 | | | Del. |
| 7966690 | TBX3 | 12 | 2.29 | 1.39 | 1.58 | 1.714 | | | Amp. |
| 7899615 | SERINC2 | 1 | 2.44 | 2.13 | 2.37 | 2.312 | | Amp. | |
| 8049349 | UGT1A | 2 | 1.28 | -0.11 | 0.17 | 0.288 | | | |
| 8106986 | RHOBTB3 | 5 | -1.64 | 0.15 | -3 | -0.91 | | | |
| 8027748 | FXYD3 | 19 | 3.4 | 1.02 | 1.88 | 1.868 | | | |
| 7973433 | DHRS2 | 14 | 0.45 | 0.87 | 2.2 | 0.95 | Del. | Del. | |
| 8101675 | ABCG2 | 4 | 2.87 | 1.01 | 0.27 | 0.922 | | | |
| 8151730 | CALB1 | 8 | 3.44 | 0.8 | 1.74 | 1.683 | | | |
| 7927215 | ALOX5 | 10 | 2.78 | 0.73 | 1.59 | 1.479 | | | |
| 8045889 | TANC1 | 2 | 1.68 | 0.3 | 0.33 | 0.552 | | | |
| 7925531 | AKT3 | 1 | 1.98 | 0.91 | 2.19 | 1.578 | | Amp. | |
| 8098441 | ODZ3 | 4 | 1.57 | 0.28 | 1.61 | 0.896 | | | Del. |
| 8044574 | IL1RN | 2 | 1.81 | 0.1 | 0.24 | 0.354 | | Del. | |
| 8038683 | KLK6 | 19 | 3.25 | 0.93 | 0.87 | 1.381 | | Amp. | |
| 7922773 | NCF2 | 1 | 1.59 | 0.09 | 0.65 | 0.454 | | | |
| 8068100 | NCRNA00189 | 21 | 0.11 | 0.29 | 1.35 | 0.347 | | | Amp. |
| 8037205 | CEACAM1 | 19 | 3.05 | 0.75 | 1.64 | 1.556 | | Amp. | |
| 7918657 | PTPN22 | 1 | 3.67 | 1.53 | 0.72 | 1.591 | | | |
| 8098263 | PALLD | 4 | -1.96 | -1.72 | -2.27 | -1.97 | | | Del. |
| 8053417 | CAPG | 2 | 1.43 | -0.7 | -0.23 | 0.616 | | Amp. | |
| 8016457 | HOXB5 | 17 | 1.49 | 1.97 | 2.44 | 1.927 | | | |
| 8067055 | ATP9A | 20 | 1.07 | 0.04 | -0.64 | 0.301 | | | |
| 7902104 | PDE4B | 1 | -2.32 | -0.11 | -2.07 | -0.8 | | | |
| 8077899 | PPARG | 3 | 2.26 | 0.56 | 0.56 | 0.89 | | | |
| 8015016 | TNS4 | 17 | 0.52 | 0.83 | 1.68 | 0.895 | | | |
| 7915472 | SLC2A1 | 1 | -1.73 | 0.8 | 1.04 | 1.13 | | | |
| 8095728 | EREG | 4 | -1.52 | 0.1 | -3.87 | -0.83 | | | |
| 7923958 | C1orf116 | 1 | 2.01 | 0.54 | 0.82 | 0.96 | | | |
| 7955694 | IGFBP6 | 12 | 2.27 | 1.12 | 1.5 | 1.56 | | | |
| 8112803 | LHFPL2 | 5 | 1.39 | 0.1 | -0.15 | 0.273 | | | |
| 8033780 | ZNF426 | 19 | -1.11 | 1.12 | -0.92 | -1.04 | | | |
| 8016463 | HOXB6 | 17 | 1.53 | 2.06 | 2.45 | 1.979 | | | |
| 7940643 | ASRGL1 | 11 | -1.35 | 0.56 | 0.01 | -0.2 | | Amp. | |
| 7961182 | KLRC2 | 12 | -3.17 | -0.99 | -1.91 | -1.82 | | | Amp. |
| 8038695 | KLK7 | 19 | 2.78 | 0.72 | 0.82 | 1.178 | | Amp. | |
| 7950534 | WNT11 | 11 | 2.45 | 0.77 | 0.45 | 0.951 | Amp. | Amp. | |
| 7986214 | SLCO3A1 | 15 | 2.27 | 0.53 | 1.26 | 1.148 | | | |
| 8098246 | ANXA10 | 4 | -0.19 | -1.75 | -1.4 | -0.77 | | | |
| 7990391 | CYP1A1 | 15 | 2.51 | 1.14 | 0.91 | 1.374 | | | |
| 7946781 | PLEKHA7 | 11 | 1.68 | 0.52 | 0.43 | 0.722 | Amp. | Amp. | |
| 8070411 | C21 orf88 | 21 | 1.43 | -0.21 | 0.11 | 0.32 | | | Amp. |
| 7920128 | S100A11 | 1 | 1.24 | 0.69 | 1.6 | 1.108 | | Amp. | |
| 7902594 | PRKACB | 1 | -3.7 | -2.59 | -3.14 | -3.11 | | | |
| 7957023 | LYZ | 12 | 3.63 | 0.7 | 1.24 | 1.466 | | | |
| 8150509 | PLAT | 8 | 1.92 | -0.61 | 0.77 | 0.968 | | | |
| 7920285 | S100A2 | 1 | 1.43 | -0.12 | -7.87E-05 | 0.024 | | Amp. | |
| 7976425 | OTUB2 | 14 | 1.56 | 0.69 | 0.81 | 0.957 | Del. | | |
| 8122146 | nd | 6 | -2.21 | 0.89 | 0.2 | -0.74 | | | |
| 8042993 | CTNNA2 | 2 | 1.1 | -0.03 | 0.33 | 0.227 | | | |
| 8076497 | A4GALT | 22 | 1.39 | 1 | 2.15 | 1.439 | | Amp. | |
| 8073068 | APOBEC3C | 22 | 1.82 | 1.35 | 1.77 | 1.633 | | Amp. | |
| 7917850 | ARHGAP29 | 1 | -4.1 | -1.54 | -1.73 | -2.22 | | | |
| 7938035 | TRIM22 | 11 | 1.04 | 1.76 | 0.49 | 0.964 | Amp. | | |
| 7963333 | KRT80 | 12 | 1.51 | -0.15 | -0.03 | 0.199 | | | |
| 7932985 | NRP1 | 10 | 2.95 | -0.18 | 0.18 | 0.458 | | | |
| 7961151 | KLRK1 | 12 | -4.33 | -0.91 | -2.15 | -2.04 | | | Amp. |
| 7899627 | TINAGL1 | 1 | 1.57 | 0.95 | 1.65 | 1.348 | | Amp. | |

**Table 4. Top 100 differentially expressed genes (Cumulative p-value <0.001) and corresponding copy number changes in HCT116 p53-/-: CYC116 group.**

| Gene ID | Gene symbol | Chr. | R2.1 logFC | R2.2 logFC | R2.3 logFC | IogFC Mean | R2.1 Copy No. | R2.2 Copy No. | R2.3 Copy No. |
|---|---|---|---|---|---|---|---|---|---|
| 8135763 | WNT16 | 7 | -0.6 | -3.9 | -0.38 | -0.95 | | | |
| 7906954 | PBX1 | 1 | 1.38 | 4.11 | 1.36 | 1.98 | | | |
| 8140955 | CDK6 | 7 | -2.05 | 1.29 | -1.69 | -1.65 | | Amp. | |
| 8171297 | MID1 | X | -3.99 | -4 | -4.66 | -4.19 | Del. | Del. | Del. |
| 7939314 | EHF | 11 | 5.37 | 1.13 | 4.74 | 3.07 | | | |
| 8013384 | ALDH3A1 | 17 | 0.5 | 3.72 | 0.24 | 0.76 | | | Del. |
| 8046726 | SSFA2 | 2 | -0.47 | -2.1 | -0.51 | -0.8 | Del. | | Del. |
| 8152376 | CSMD3 | 8 | -0.3 | 1.67 | -0.12 | 0.39 | | Del. | |
| 8067140 | CYP24A1 | 20 | -5.54 | -3.7 | -5.79 | -4.92 | | | |
| 8140468 | PION | 7 | 4.09 | -0.2 | 3.51 | 1.44 | | | |
| 7895417 | SEPT2 | 2 | -1.83 | -0.1 | -2.04 | -0.6 | | | |
| 8106727 | ATP6AP1 L | 5 | 2.49 | -0.2 | 2.26 | 1.01 | Amp. | Amp. | Amp. |
| 7951686 | IL18 | 11 | 0.6 | -1.7 | 0.58 | -0.84 | Amp. | Amp. | Amp. |
| 8148309 | Nd | 8 | -1.39 | -1.7 | -1.18 | -1.42 | | Del. | |
| 8140668 | SEMA3A | 7 | 0.48 | -2.5 | 0.56 | -0.87 | | | |
| 8081548 | PVRL3 | 3 | -0.51 | -2.4 | -0.6 | -0.9 | | Amp. | |
| 7950810 | SYTL2 | 11 | 1.44 | -1.6 | 1.2 | 1.42 | Amp. | Amp. | Amp. |
| 7910915 | CHRM3 | 1 | -0.19 | 2.02 | 0.13 | 0.37 | | Del. | |
| 8038695 | KLK7 | 19 | 1.48 | 0.1 | 1.61 | 0.61 | | | |
| 7917850 | ARHGAP29 | 1 | -1.95 | -3.9 | -1.25 | -2.11 | | | |
| 8113761 | ZNF608 | 5 | -1 | -1.7 | -0.98 | -1.19 | Amp. | Amp. | Amp. |
| 8076497 | A4GALT | 22 | 0.89 | 1.68 | 1.1 | 1.18 | | | |
| 8122634 | SAMD5 | 6 | 2 | -0.3 | 1.6 | 1 | | | |
| 7957298 | NAV3 | 12 | -0.04 | -2 | 0.11 | -0.21 | | | |
| 8073096 | APOBEC3H | 22 | 1.71 | 0.86 | 1.84 | 1.39 | | | |
| 8114119 | FSTL4 | 5 | 1.54 | 1.3 | 1.58 | 1.47 | Amp. | | Amp. |
| 7958884 | OAS1 | 12 | 0.3 | 2.31 | 0.37 | 0.64 | | | |
| 8121749 | GJA1 | 6 | 0.25 | -0 | 1.86 | 0.28 | Amp. | Amp. | Amp. |
| 7965941 | GLT8D2 | 12 | 0.94 | -0.8 | 0.88 | 0.86 | | | |
| 8141066 | PON3 | 7 | -2.23 | -2.2 | -1.95 | -2.11 | | | |
| 7906969 | Nd | 1 | 0.05 | 1.85 | 0.13 | 0.23 | | | |
| 8023043 | PSTPIP2 | 18 | -0.01 | -1.3 | -0.24 | -0.15 | Amp. | Del. | |
| 8097356 | PLK4 | 4 | -1.31 | -0.8 | -1.42 | -1.16 | Del. | Del. | Del. |
| 7962151 | DENND5B | 12 | 0.96 | 1.65 | 0.86 | 1.11 | | | |
| 7932744 | ARMC4 | 10 | -0.38 | -1.9 | -0.33 | -0.62 | | | |
| 7934161 | PRF1 | 10 | -2.9 | -2.2 | -2.8 | -2.63 | Amp. | Amp. | Amp. |
| 8127234 | DST | 6 | -1.27 | -2.2 | -1.36 | -1.57 | Amp. | Amp. | Amp. |
| 8084630 | Nd | 3 | 1.37 | 2.24 | 1.15 | 1.52 | | Amp. | |
| 8084630 | Nd | 3 | 1.37 | 2.24 | 1.15 | 1.52 | | Amp. | |
| 8084630 | Nd | 3 | 1.37 | 2.24 | 1.15 | 1.52 | | Amp. | |
| 8007446 | IFI35 | 17 | -0.46 | 2.23 | -0.45 | 0.77 | | | |
| 8115490 | ADAM19 | 5 | 0.68 | -2 | 0.4 | -0.81 | | | |
| 8082075 | DTX3L | 3 | -0.45 | 1.39 | -0.12 | 0.42 | | Amp. | |
| 8075310 | LIF | 22 | 1.3 | -0.2 | 1.35 | 0.66 | | | |
| 8102950 | INPP4B | 4 | -0.68 | -2.7 | -1.01 | -1.23 | Del. | Del. | Del. |
| 8027748 | FXYD3 | 19 | 0.74 | 2.71 | 0.76 | 1.15 | | | |
| 8065071 | FLRT3 | 20 | 0.34 | 1.64 | 0.21 | 0.49 | | | |
| 8101828 | TSPAN5 | 4 | -1.08 | -2.8 | -1.11 | -1.49 | Del. | Del. | Del. |
| 8166747 | SYTL5 | X | 0.85 | -2.4 | 0.9 | -1.22 | | | |
| 7990391 | CYP1A1 | 15 | 2.56 | 4.74 | 2.21 | 2.99 | | | Amp. |
| 8152506 | SAMD12 | 8 | 1.51 | 1.81 | 1.63 | 1.64 | | Del. | Del. |
| 7927202 | ZN F22 | 10 | -2.48 | -2 | -2.29 | -2.23 | Amp. | Amp. | Amp. |
| 7902594 | PRKACB | 1 | -1.56 | -2 | -1.35 | -1.62 | Amp. | Amp. | Amp. |
| 8036318 | ZNF566 | 19 | -0.68 | 1.35 | -0.8 | -0.9 | | Del. | |
| 7935521 | AVPI1 | 10 | 1.08 | 1.17 | 1.19 | 1.15 | Amp. | Amp. | Amp. |
| 8022711 | DSC2 | 18 | -0.02 | -1.5 | -0.34 | -0.22 | Amp. | Del. | Amp. |
| 7932765 | MPP7 | 10 | -0.12 | -1.4 | -0.17 | -0.3 | | Del. | Del. |
| 7957260 | GLIPR1 | 12 | -0.81 | -2.7 | -0.48 | -1.01 | | | |
| 7916862 | WLS | 1 | 1.12 | -0.6 | 1.21 | 0.93 | | | |
| 8102415 | CAMK2D | 4 | -0.66 | -1.7 | -0.77 | -0.95 | Del. | Del. | Del. |
| 8150830 | LYPLA1 | 8 | -1.23 | -1.1 | -1.07 | -1.12 | Del. | Del. | Del. |
| 8154135 | SLC1A1 | 9 | 1.03 | -1.8 | 0.97 | 1.21 | Amp. | Del. | |
| 8148304 | TRIB1 | 8 | 0.03 | -0.9 | 0.23 | -0.18 | | Del. | |
| 8106743 | VCAN | 5 | 1.05 | -2.6 | 1.14 | -1.47 | Amp. | Amp. | Amp. |
| 8005029 | MAP2K4 | 17 | -1.2 | -0.6 | -1.38 | -1.01 | Del. | | Del. |
| 8138566 | IGF2BP3 | 7 | -2.63 | -0.3 | -1.63 | -1.05 | | Amp. | |
| 8059716 | C2orf52 | 2 | 1.18 | 0.75 | 1.54 | 1.11 | Amp. | Amp. | Amp. |
| 8106986 | RHOBTB3 | 5 | -0.41 | -2 | -0.54 | -0.76 | Amp. | Amp. | Amp. |
| 8016094 | GJC1 | 17 | -2.55 | -1.9 | -2.36 | -2.24 | Amp. | Amp. | |
| 8133018 | ZNF716 | 7 | 0.05 | 2.51 | 0.53 | 0.39 | Amp. | Amp. | Amp. |
| 8144758 | ZDHHC2 | 8 | 0.41 | -0.8 | 0.45 | 0.53 | Del. | Del. | Del. |
| 8129482 | SAMD3 | 6 | -0.07 | -1.2 | -0.1 | -0.2 | Amp. | | |
| 7917528 | Nd | 1 | -0.34 | 0.6 | -0.68 | -0.52 | | | |
| 8100328 | USP46 | 4 | -0.84 | 0.11 | -0.85 | -0.43 | Del. | Amp. | Del. |
| 8047738 | NRP2 | 2 | -0.01 | 1.1 | 0.34 | 0.17 | | Amp. | |
| 7947230 | BDNF | 11 | -0.29 | -2.2 | -0.35 | -0.6 | | | |
| 8081214 | GPR15 | 3 | 1.42 | -1.3 | 1.03 | 1.23 | | Amp. | |
| 8104107 | TRIML2 | 4 | -1.78 | -2 | -1.6 | -1.78 | | | |
| 7892605 | SEPT2 | 2 | -1.5 | 0.12 | -1.33 | -0.62 | | | |
| 8120176 | C6orf141 | 6 | 0.27 | -1.2 | 0.64 | -0.59 | Amp. | Amp. | Amp. |
| 7930498 | ACSL5 | 10 | -1.7 | -2 | -1.18 | -1.59 | | | |
| 8060225 | HDLBP | 2 | -0.91 | -0.1 | -1.07 | -0.38 | | Amp. | Amp. |
| 8152617 | HAS2 | 8 | 2.11 | 0.03 | 2.25 | 0.53 | | Del. | Del. |
| 7935660 | DNMBP | 10 | -0.34 | -1.7 | -0.44 | -0.64 | Amp. | | |
| 8075910 | RAC2 | 22 | -0.01 | -1.2 | -0.06 | -0.08 | | | |
| 8059345 | SCG2 | 2 | -1.05 | 0.23 | -1.16 | -0.65 | | Amp. | |
| 8081158 | ARL6 | 3 | -0.24 | 0.98 | -0.09 | 0.27 | | Amp. | |
| 8035095 | CYP4F11 | 19 | -1.87 | -0.7 | -2.06 | -1.36 | | | Amp. |
| 8160670 | AQP3 | 9 | 0.41 | 2.75 | 0.25 | 0.65 | | | |
| 8141035 | SGCE | 7 | -1.18 | 0.39 | -0.64 | -0.67 | | | |
| 8059111 | ABCB6 | 2 | -0.21 | 0.74 | -0.34 | 0.37 | | Amp. | Amp. |
| 8059111 | ATG9A | 2 | -0.21 | 0.74 | -0.34 | 0.37 | | Amp. | Amp. |
| 7988260 | FRMD5 | 15 | -1.5 | -1.7 | -1.38 | -1.52 | Amp. | | Amp. |
| 7896498 | SEPT2 | 2 | -0.81 | -0 | -1.07 | -0.33 | | | |
| 8017651 | SMURF2 | 17 | -1.08 | -1 | -1.14 | -1.06 | Amp. | | |
| 8146379 | UBE2V2 | 8 | -0.81 | -0.5 | -0.92 | -0.71 | Del. | Del. | Del. |
| 7993478 | ABCC1 | 16 | -0.2 | 1.12 | -0.17 | 0.33 | | Amp. | |
| 8017843 | SLC16A6 | 17 | 2.4 | -0.6 | 2.61 | 1.6 | | | |
| 8112615 | ENC1 | 5 | 0.09 | -1.5 | 0.39 | -0.38 | Amp. | Amp. | Amp. |
| 7902553 | IFI44 | 1 | 1.36 | 2.39 | 0.89 | 1.43 | | | |

**Table 5. Top 100 differentially expressed genes (Cumulative p-value <0.001) and corresponding copy number changes in HCT116: ZM447439 group.**

| **Gene ID** | **Gene symbol** | **Chr.** | **R3.1 logFC** | **R3.2 logFC** | **R3.3 logFC** | **logFC Mean** | **R3.1 Copy No.** | **R3.2 Copy No.** | **R3.3 Copy No.** |
|---|---|---|---|---|---|---|---|---|---|
| 8098441 | ODZ3 | 4 | 1.949 | 1.872 | 2.185 | 1.998 | Del. | | |
| 7932744 | ARMC4 | 10 | -2.59 | -2.67 | -2.52 | -2.59 | Amp. | | |
| 8144726 | TUSC3 | 8 | 1.872 | 2.211 | 2.602 | 2.209 | Amp. | | |
| 8098263 | PALLD | 4 | -2.18 | -2 | -1.99 | -2.05 | Amp. | | |
| 7989146 | MNS1 | 15 | -1.61 | -1.56 | -1.35 | -1.5 | | | |
| 7894805 | Nd | 1 | -0.43 | -1.91 | -0.55 | -0.77 | | | |
| 8021169 | LIPG | 18 | -1.03 | -1 | -1.22 | -1.08 | | | |
| 8059854 | ARL4C | 2 | 1.866 | 0.953 | 1.152 | 1.27 | | | |
| 7893924 | Nd | 5 | 4.604 | 6.218 | 5.593 | 5.43 | | | |
| 7895294 | ILF2 | 1 | -1.37 | -1.33 | -0.49 | -0.96 | | | |
| 8122176 | TCF21 | 6 | -1.22 | -0.97 | -1.06 | -1.08 | | | |
| 7932765 | MPP7 | 10 | -2.08 | -2.28 | -2.2 | -2.18 | Amp. | | |
| 7895205 | Nd | 1 | 1.628 | 1.559 | 1.57 | 1.586 | | | |
| 7894487 | Nd | 2 | -1.06 | -1.46 | -0.28 | -0.75 | | | |
| 7893953 | Nd | 17 | 0.941 | 1.278 | 1.175 | 1.122 | | | |
| 7975154 | NCRNA00238 | 14 | 1.573 | 0.154 | 0.215 | 0.373 | Del. | | |
| 7896206 | Nd | 14 | -0.39 | -1.42 | -0.71 | -0.73 | | | |
| 7932733 | MKX | 10 | -1.76 | -1.68 | -1.75 | -1.73 | Amp. | | |
| 8152376 | CSMD3 | 8 | 1.521 | 1.813 | 1.934 | 1.747 | Amp. | | |
| 8112615 | ENC1 | 5 | -1.86 | -1.39 | -0.99 | -1.37 | Amp. | | |
| 8102328 | CFI | 4 | 0.822 | 0.178 | 0.071 | 0.218 | Del. | | |
| 8088952 | Nd | 3 | 1.552 | 0.431 | 0.654 | 0.759 | | | |
| 7893175 | Nd | 19 | 1.829 | 1.995 | 1.755 | 1.857 | | | |
| 8089467 | ZBED2 | 3 | -1.75 | -0.71 | -0.47 | -0.83 | Amp. | Amp. | |
| 8013519 | Nd | 17 | 1.872 | 1.107 | 0.327 | 0.878 | | | |
| 8013519 | Nd | 5 | 1.872 | 1.107 | 0.327 | 0.878 | | | |
| 8003230 | Nd | 16 | 0.991 | 0.934 | 1.073 | 0.998 | Del. | | |
| 7899615 | SERINC2 | 1 | 0.523 | 1.289 | 1.146 | 0.917 | Del. | | |
| 7937335 | IFITM...fg | 11 | 2.179 | 0.229 | 0.228 | 0.484 | Del. | | |
| 7937335 | IFITM1 | 11 | 2.179 | 0.229 | 0.228 | 0.484 | Del. | | |
| 7937335 | IFITM2 | 11 | 2.179 | 0.229 | 0.228 | 0.484 | Del. | | |
| 7934731 | C1DP...fg | 10 | 0.217 | -0.9 | -1.12 | -0.6 | | | |
| 7934731 | C1DP2 | 10 | 0.217 | -0.9 | -1.12 | -0.6 | | | |
| 7934731 | C1DP3 | 10 | 0.217 | -0.9 | -1.12 | -0.6 | | | |
| 7934731 | C1DP1 | 10 | 0.217 | -0.9 | -1.12 | -0.6 | | | |
| 7934731 | C1DP4 | 10 | 0.217 | -0.9 | -1.12 | -0.6 | | | |
| 7934731 | C1D | 2 | 0.217 | -0.9 | -1.12 | -0.6 | | | |
| 7903717 | MIR197 | 1 | 0.687 | 1.372 | 1.049 | 0.996 | | | |
| 7952205 | MCAM | 11 | 0.958 | 0.824 | 0.882 | 0.886 | Del. | | |
| 7894185 | OAZ1 | 19 | -0.71 | -1.08 | -0.69 | -0.81 | | | |
| 8142763 | Nd | 7 | -0.73 | -0.58 | 0.019 | -0.2 | Del. | | |
| 7947230 | BDNF | 11 | -1.14 | -1.57 | -1.3 | -1.32 | Del. | Del. | Del. |
| 8135594 | CAV1 | 7 | -1.17 | -1.22 | -1.38 | -1.26 | | | |
| 7902265 | Nd | 1 | 0.946 | 1.285 | 1.087 | 1.098 | | | |
| 7901175 | TSPAN1 | 1 | 1.563 | 1.468 | 1.121 | 1.37 | Del. | | |
| 7916493 | PPAP2B | 1 | 0.755 | 0.616 | 0.514 | 0.621 | Amp. | | |
| 7894891 | Nd | 2 | 1.25 | 2.188 | 1.987 | 1.758 | | | |
| 7893711 | ABCF1 | 6 | 1.828 | 1.907 | 1.65 | 1.792 | | | |
| 7995320 | Nd | 16 | 1.188 | 1.597 | 1.266 | 1.339 | Amp. | | |
| 7995320 | Nd | 16 | 1.188 | 1.597 | 1.266 | 1.339 | Amp. | | |
| 7995320 | Nd | 16 | 1.188 | 1.597 | 1.266 | 1.339 | Amp. | | |
| 7995320 | Nd | 16 | 1.188 | 1.597 | 1.266 | 1.339 | Amp. | | |
| 7895508 | Nd | 6 | 0.357 | 0.815 | 0.685 | 0.584 | | | |
| 8155497 | FAM27C | 9 | 1.575 | 1.948 | 1.795 | 1.766 | Amp. | | |
| 7921987 | TMCO1 | 1 | -0.6 | -0.88 | -0.61 | -0.69 | Del. | | |
| 8083453 | Nd | 17 | 0.612 | 0.832 | 0.776 | 0.734 | | | |
| 8083453 | Nd | 17 | 0.612 | 0.832 | 0.776 | 0.734 | | | |
| 8083453 | Nd | 17 | 0.612 | 0.832 | 0.776 | 0.734 | | | |
| 8083453 | Nd | 17 | 0.612 | 0.832 | 0.776 | 0.734 | | | |
| 8083453 | .nd | 2 | 0.612 | 0.832 | 0.776 | 0.734 | | | |
| 8083453 | Nd | 2 | 0.612 | 0.832 | 0.776 | 0.734 | | | |
| 8083453 | Nd | 2 | 0.612 | 0.832 | 0.776 | 0.734 | | | |
| 8083453 | Nd | 2 | 0.612 | 0.832 | 0.776 | 0.734 | | | |
| 8083453 | Nd | 3 | 0.612 | 0.832 | 0.776 | 0.734 | | | |
| 8083453 | Nd | 3 | 0.612 | 0.832 | 0.776 | 0.734 | | | |
| 8083453 | Nd | 3 | 0.612 | 0.832 | 0.776 | 0.734 | | | |
| 8111255 | CDH10 | 5 | 0.53 | 0.763 | 0.896 | 0.713 | | Amp. | |
| 7896217 | Nd | 19 | -0.35 | -1.17 | -0.48 | -0.58 | | | |
| 8132962 | CCT6A | 7 | -0.04 | -2.01 | -0.52 | -0.35 | Del. | | |
| 8132962 | SNORA15 | 7 | -0.04 | -2.01 | -0.52 | -0.35 | Del. | | |
| 7893844 | Nd | 14 | 0.813 | 1.207 | 0.819 | 0.93 | | | |
| 8044080 | SLC9A2 | 2 | -0.85 | -0.7 | -0.73 | -0.76 | Amp. | | |
| 8130499 | DYNLT1 | 6 | -0.83 | -1.05 | -1.02 | -0.96 | Del. | Del. | |
| 8065082 | Nd | 20 | -0.54 | 0.106 | -0.26 | -0.25 | | | |
| 8106923 | NR2F1 | 5 | -0.87 | -0.73 | -0.89 | -0.83 | Del. | | |
| 8097256 | FGF2 | 4 | 0.977 | 1.204 | 1.078 | 1.083 | | | |
| 8144667 | SUB1P1 | 8 | -0.68 | -1.04 | -0.79 | -0.83 | Del. | | |
| 8082607 | ATP2C1 | 3 | -0.86 | -0.97 | -0.85 | -0.89 | Del. | | |
| 7895711 | Nd | 2 | 1.345 | -0.05 | 0.307 | 0.282 | | | |
| 7912994 | IFFO2 | 1 | 1.219 | 0.709 | 0.66 | 0.829 | Del. | | |
| 7925531 | AKT3 | 1 | 1.595 | 1.035 | 1.077 | 1.212 | Amp. | Del. | |
| 7893864 | Nd | 6 | 0.227 | -0.68 | -0.55 | -0.44 | | | |
| 7971669 | Nd | 13 | 0.7 | 1.23 | 0.983 | 0.946 | Del. | Del. | Del. |
| 7895521 | HNRNPD | 4 | -0.61 | -0.74 | -0.29 | -0.51 | | | |
| 7896540 | Nd | 12 | 1.524 | 1.961 | 1.978 | 1.808 | | | |
| 8079426 | TMIE | 3 | 0.318 | 0.756 | 0.443 | 0.474 | Del. | | |
| 7895791 | Nd | 19 | -0.69 | -1.01 | -0.15 | -0.47 | | | |
| 7896112 | Nd | 2 | -0.55 | -1.16 | -0.31 | -0.58 | | | |
| 7896112 | IK | 5 | -0.55 | -1.16 | -0.31 | -0.58 | | | |
| 7892996 | Nd | 2 | 0.13 | -0.82 | -0.44 | -0.36 | | | |
| 7892996 | Nd | 5 | 0.13 | -0.82 | -0.44 | -0.36 | | | |
| 8114396 | CDC23 | 5 | -0.69 | -1.1 | -0.67 | -0.8 | Del. | | |
| 8100376 | Nd | 4 | 0.755 | 0.991 | 0.717 | 0.813 | Amp. | | |
| 7893051 | Nd | 5 | 1.731 | 2.423 | 2.256 | 2.115 | | | |
| 8109424 | Nd | 5 | 1.109 | 1.602 | 1.549 | 1.402 | | | |
| 8105612 | CWC27 | 5 | -0.66 | -0.92 | -0.73 | -0.76 | Amp. | | |
| 7905444 | SNX27 | 1 | -0.49 | -0.68 | -0.52 | -0.56 | | | |
| 8052370 | Nd | 2 | 0.843 | 1.339 | 0.915 | 1.011 | Amp. | | |
| 8098246 | ANXA10 | 4 | -1.49 | -1.67 | -1.5 | -1.55 | Amp. | | |
| 7895085 | SMNDC1 | 10 | 0.287 | -0.72 | -0.84 | -0.56 | | | |

**Table 6. Top 100 differentially expressed genes (Cumulative p-value <0.001) and corresponding copy number changes in HCT116 p53-/-: ZM447439 group.**

| **Gene ID** | **Gen symbol** | **Chr.** | **R4.1 logFC** | **R4.2 logFC** | **R4.3 logFC** | **logFC Mean** | **R4.1 Copy No.** | **R4.2 Copy No.** | **R4.3 Copy No.** |
|---|---|---|---|---|---|---|---|---|---|
| 8148040 | MAL2 | 8 | -5.55 | -5.56 | -5.68 | -5.6 | | | |
| 8067140 | CYP24A1 | 20 | -5.5 | -5.61 | -6.22 | -5.77 | | | |
| 8148280 | SQLE | 8 | -2.41 | -2.77 | -2.47 | -2.55 | | | |
| 8030804 | CD33 | 19 | 1.24 | 1.81 | 1.768 | 1.586 | Amp. | | Amp. |
| 7983650 | SLC27A2 | 15 | -3.43 | -3.35 | -2.95 | -3.24 | | | |
| 7960143 | ZNF84 | 12 | 0.19 | -1.85 | -0.5 | -0.56 | | | |
| 8113512 | EPB41L4A | 5 | 2.47 | 2.06 | 2.797 | 2.421 | | Amp. | |
| 8055496 | LRP1B | 2 | 2.02 | 0.89 | 2.048 | 1.544 | Amp. | Amp. | Amp. |
| 8135763 | WNT16 | 7 | -0.33 | -1.45 | -1.41 | -0.88 | | | |
| 8129476 | C6orf191 | 6 | 0.67 | 0.83 | 2.264 | 1.076 | | | |
| 8098246 | ANXA10 | 4 | -1.82 | -1.3 | -1.2 | -1.42 | | | |
| 7916862 | WLS | 1 | 0.91 | 0.94 | 1.253 | 1.025 | | | |
| 8135587 | CAV2 | 7 | -1.53 | -1.2 | -1.53 | -1.41 | | | |
| 8172158 | CASK | X | -2.04 | -2.02 | -1.96 | -2.01 | | | Del. |
| 8023561 | LMAN1 | 18 | -3.1 | -3.36 | -3.05 | -3.17 | | Amp. | Amp. |
| 7901175 | TSPAN1 | 1 | 0.72 | 1.65 | 0.988 | 1.054 | | | |
| 8036318 | ZNF566 | 19 | 1.19 | -0.44 | 1.368 | 0.893 | | | |
| 7961166 | KLRC4 | 12 | 0.38 | -0.72 | 1.128 | 0.677 | | | |
| 8115327 | SPARC | 5 | 2.8 | 2.76 | 2.87 | 2.809 | | | |
| 8148309 | Nd | 8 | -1.33 | -2 | -1.34 | -1.53 | | | |
| 8103415 | FAM198B | 4 | 0.96 | 1.29 | 2.959 | 1.544 | | | |
| 8028058 | KIRREL2 | 19 | 1.54 | 1.43 | 1.52 | 1.494 | | | |
| 8135594 | CAV1 | 7 | -2.22 | -1.89 | -2.22 | -2.1 | | | |
| 8151496 | ZNF704 | 8 | 1.4 | 1.03 | 1.118 | 1.174 | | | |
| 8102415 | CAMK2D | 4 | -1.59 | -1.38 | -1.54 | -1.5 | Del. | | |
| 8038192 | FUT1 | 19 | 0.58 | 1.2 | 0.358 | 0.629 | | | |
| 8166747 | SYTL5 | X | -1.53 | -1.63 | -2.13 | -1.74 | | | |
| 8106986 | RHOBTB3 | 5 | -0.86 | -1.59 | -0.8 | -1.03 | | | |
| 7977933 | SLC7A8 | 14 | 1.27 | 1.11 | 1.885 | 1.385 | Amp. | | Amp. |
| 7902104 | PDE4B | 1 | -1.56 | -1.81 | -1.36 | -1.57 | | | |
| 8003060 | SDR42E1 | 16 | -1.4 | -1.46 | -1.2 | -1.35 | | | |
| 7954559 | PPFIBP1 | 12 | 0.14 | -1.05 | 0.143 | -0.28 | | | |
| 8138805 | CPVL | 7 | 1.11 | 0.64 | 0.932 | 0.872 | | | |
| 8180200 | ZNF493 | 19 | -0.77 | -0.72 | -1.11 | -0.85 | | | |
| 7934970 | HTR7 | 10 | -1.28 | -1.21 | -1.59 | -1.35 | | | |
| 7932744 | ARMC4 | 10 | 0.23 | -0.9 | 0.348 | -0.42 | | | |
| 8072587 | SLC5A1 | 22 | 0.34 | 0.75 | 1.506 | 0.73 | | | |
| 8096160 | ARHGAP24 | 4 | 1.26 | 1.28 | 1.282 | 1.276 | Del. | | |
| 7982066 | Nd | 15 | -0.12 | 2.09 | 0.734 | 0.568 | Amp. | | Amp. |
| 7982066 | SNORD115-24 | 15 | -0.12 | 2.09 | 0.734 | 0.568 | Amp. | | Amp. |
| 7982066 | SNORD115-30 | 15 | -0.12 | 2.09 | 0.734 | 0.568 | Amp. | | Amp. |
| 7982066 | SNORD115-42 | 15 | -0.12 | 2.09 | 0.734 | 0.568 | Amp. | | Amp. |
| 7978376 | STXBP6 | 14 | -0.66 | 0.06 | -0.88 | -0.33 | Amp. | Amp. | Amp. |
| 8127563 | COL12A1 | 6 | -0.83 | -1.61 | -1.24 | -1.18 | | Amp. | |
| 8035847 | ZNF675 | 19 | -0.62 | -1.4 | -0.5 | -0.76 | Amp. | | Amp. |
| 8069880 | TIAM1 | 21 | -0.88 | -0.8 | -1.03 | -0.9 | | | |
| 8126820 | GPR110 | 6 | -0.4 | -1.56 | 0.481 | -0.67 | | | |
| 8040163 | IAH1 | 2 | -0.86 | -0.89 | -0.99 | -0.91 | | | |
| 8099393 | Nd | 4 | -1.23 | -0.22 | -0.75 | -0.58 | | Amp. | |
| 7926875 | BAMBI | 10 | 0.42 | 1.32 | 1.625 | 0.964 | | | |
| 8081214 | GPR15 | 3 | -1.24 | -1.54 | -1.3 | -1.36 | | | |
| 8167973 | HEPH | X | 1.31 | 0.76 | 0.814 | 0.933 | | | |
| 8110084 | MSX2 | 5 | -1.49 | -1.35 | -1.44 | -1.43 | | | |
| 8174527 | CAPN6 | X | 0.96 | 0.68 | 1.222 | 0.929 | | | |
| 7943263 | AMOTL1 | 11 | 0.29 | -0.79 | -0.05 | -0.23 | | | |
| 8149927 | CLU | 8 | -0.43 | -0.66 | -0.73 | -0.59 | | | |
| 8085263 | TMEM111 | 3 | -1.23 | -1.27 | -1.3 | -1.26 | | | |
| 7960134 | ZNF26 | 12 | -1.58 | -1.82 | -1.32 | -1.56 | | | |
| 8175217 | GPC4 | X | -0.5 | 0.77 | 0.551 | 0.595 | | | |
| 7951077 | SESN3 | 11 | -1.87 | -1.9 | -1.31 | -1.67 | | | |
| 8117045 | RBM24 | 6 | 0.32 | -1.09 | -0.22 | -0.43 | Amp. | | Amp. |
| 8053325 | Nd | 2 | 0.34 | 0.99 | 1.27 | 0.754 | | | |
| 7961175 | KLRC3 | 12 | -0.09 | -0.79 | 0.38 | -0.3 | | | |
| 8168749 | SRPX2 | X | -0.93 | -0.89 | -1.23 | -1 | | | |
| 7932765 | MPP7 | 10 | 0.07 | -1.14 | -0.2 | -0.26 | Del. | Del. | Del. |
| 8060988 | BTBD3 | 20 | 1.37 | 1.16 | 1.154 | 1.222 | | | |
| 8049487 | MLPH | 2 | -1.17 | -1.22 | -1.38 | -1.25 | Amp. | Amp. | Amp. |
| 8035842 | ZNF91 | 19 | -0.41 | -1.51 | -1.06 | -0.87 | | | Amp. |
| 8033754 | ZNF266 | 19 | -1.4 | -1.19 | -1.22 | -1.27 | | | |
| 8062041 | ACSS2 | 20 | 0.52 | 1.22 | 0.291 | 0.568 | | | |
| 7997010 | CLEC18...fg | 16 | -0.95 | 0.29 | -1.55 | -0.75 | | | Amp. |
| 7997010 | CLEC18A | 16 | -0.95 | 0.29 | -1.55 | -0.75 | | | Amp. |
| 7997010 | CLEC18C | 16 | -0.95 | 0.29 | -1.55 | -0.75 | | | Amp. |
| 8015133 | KRT23 | 17 | -2.08 | -1.84 | -0.81 | -1.46 | Amp. | | Amp. |
| 8074853 | ZNF280A | 22 | -0.78 | -0.65 | -0.77 | -0.73 | | | |
| 7958352 | BTBD11 | 12 | 1.19 | 1.37 | 1.502 | 1.349 | | | |
| 7951686 | IL18 | 11 | -0.85 | 0.11 | -0.08 | -0.19 | | | |
| 8175269 | FAM122B | X | -0.7 | -0.6 | -0.55 | -0.61 | | | |
| 8045336 | GPR39 | 2 | 0.29 | 1.34 | -0.07 | 0.301 | Del. | Del. | Del. |
| 7960529 | SCNN1A | 12 | -0.98 | -0.23 | -1.11 | -0.63 | | | |
| 7896179 | Nd | 14 | -0.16 | -1.04 | 0.045 | -0.2 | | | |
| 8161737 | Nd | 9 | -0.74 | -1.09 | -0.64 | -0.8 | Del. | Del. | Del. |
| 8117415 | HIST1H3E | 6 | 0.65 | 0.56 | 0.808 | 0.665 | Amp. | | Amp. |
| 8145365 | DOCK5 | 8 | -0.89 | -0.46 | -0.73 | -0.67 | | | |
| 8063923 | SLCO4A1 | 20 | 1.07 | 1.14 | 0.805 | 0.995 | Amp. | | |
| 7961151 | KLRK1 | 12 | 0.42 | -0.32 | 1.368 | 0.567 | | | |
| 7893748 | Nd | 16 | -0.42 | -0 | 0.633 | 0.096 | | | |
| 8150862 | Nd | 8 | -0.78 | -0.85 | -0.86 | -0.83 | | | |
| 7951036 | SNORD5 | 11 | -0.86 | -1.07 | -0.83 | -0.91 | | | |
| 7951036 | SNORA18 | 11 | -0.86 | -1.07 | -0.83 | -0.91 | | | |
| 7951036 | MIR1304 | 11 | -0.86 | -1.07 | -0.83 | -0.91 | | | |
| 8082058 | CSTA | 3 | -0.01 | 1.55 | -0.06 | 0.083 | | | |
| 7966690 | TBX3 | 12 | 1.25 | 0.36 | 1.135 | 0.802 | Del. | Del. | Del. |
| 7894895 | ILF2 | 1 | -1.42 | -0.49 | 0.484 | -0.7 | | | |
| 8035318 | UNC13A | 19 | 0.46 | 0.83 | 0.616 | 0.618 | Amp. | | Amp. |
| 8134219 | CCDC132 | 7 | -0.83 | -0.76 | -0.5 | -0.68 | | | |
| 8106727 | ATP6AP1L | 5 | -0 | 1.25 | 0.322 | 0.12 | | | |
| 8140668 | SEMA3A | 7 | 0.83 | 0.53 | 1.002 | 0.762 | | | |
| 8103563 | DDX60 | 4 | -0.58 | -0.34 | 0.693 | -0.52 | | | |
| 8098441 | ODZ3 | 4 | -0.86 | -0.9 | -0.73 | -0.82 | | | |

### Validation of microarray based gene expression data by the qRT-PCR in CYC116 drug resistant cell lines

Top 100 common gene hits for each group were listed according to decreasing p-value. Common genes between the relevant groups, genes which were highly upregulated or downregulated, and some based on biological relevance were selected for qRT-PCR validation studies (totally 42 genes). Nearly 100% match in expression patterns was noticed between the microarray gene expression data and qRT-PCR validation. For example, Table 7 shows comparative data from global gene expression versus qRT-PCR of 12 genes further selected for validation study on CYC116 sensitive versus resistant primary tumors.

**Table 7. Relative expression trends (fold changes) between gene expression and qRT-PCR validation studies**

| **Gene** | **p53+/+: CYC116 clones** | | **p53-/-:CYC116 clones** | | **p53+/+: ZM447439 clones** | | **p53-/-: ZM447439 clones** | |
|---|---|---|---|---|---|---|---|---|
| | Microarray | qRT-PCR | Microarray | qRT-PCR | Microarray | qRT-PCR | Microarray | qRT-PCR |
| CYP24A1 | -32 | -33 | -30 | -50 | NE | NE | -55 | -200 |
| GJC1 | -3 | -3.5 | -5 | -5 | NE | NE | -1.6 | -1.4 |
| PPAP2B | 1.4 | 7 | 1.5 | 5 | 2 | 2.3 | NE | NE |
| ARHGAP29 | -5 | -5 | -4.3 | -2.3 | -2 | -2 | -2.1 | -1.1 |
| TSPAN1 | 3.2 | 3 | 2.3 | 3 | 2.6 | 2 | 2.1 | 4 |
| EHF | 5 | 32 | 8.38 | 264 | NE | NE | NE | NE |
| SEMA3A | NE | NE | -2 | 3 | NE | NE | 2 | 3 |
| KRT7 | 2 | 30 | NE | NE | NE | NE | NE | NE |
| PRKACB | -9 | -6 | -3 | -3 | -9 | -5 | NE | NE |
| ANXA 10 | -2 | -2 | -1.4 | 1.34 | -3 | -6 | -3 | -1.3 |
| SERINC2 | 5 | 7.4 | 2 | 2 | 2 | 2.1 | NE | NE |
| MID1 | -2.5 | -2 | -18 | -3 | -2 | -1.7 | NE | NE |

Fold changes of a particular gene was shown from both gene expression analysis and qRT-PCR. Positive and negative values indicate up-regulation and down-regulation of a given gene respectively. The fold change of each gene is an average value of three clones from each group. NE-not expressed

### Validation of microarray based gene expression data from the cell lines by qRT-PCR in CYC116 drug resistant primary tumor cells

Our laboratory collected various types of primary tumor biopsies and tested for CYC116 using MTT cell proliferation assay (Sargent J.M. et al., British Journal of Cancer 1989; 60, 206-10). Some samples were sensitive to CYC116 and some were resistant. 13 sensitive samples (Average IC50: ≤4.42 µM) and 14 resistant samples (Average IC50: ≥95 µM) were selected to compare gene expression towards CYC116 in resistant primary cells (Table 8). We used unselected cancers with different histogenetic origin, for instance hematological tumors (acute lymphoblastic leukemia, acute myeloid leukemia, unspecified lymphoid leukemia, Non-Hodgkins lymphoma) and solid tumors (ovarian, lung, breast, and melanoma).

**Table 8. qRT-PCR data comparing average relative Ct values of selected 12 genes in CYC116 sensitive versus resistant primary tumor samples (lower the Ct value the higher the gene expression). Primary tumor qRT-PCR data were also compared to expression trends in CYC116 resistant cell lines (▲-increased expression, ▼- decreased expression) qRT-PCR data. Data indicate perfect match of gene expression in cell lines versus primary tumors resistant to CYC116, although only subgroup genes showed significantly different expression in limited cohort of primary human tumors.**

| | **Average** | | **Trend compared to cell line data** | **p-value** |
|---|---|---|---|---|
| | **Sensitive** | **Resistant** | | |
| **CYP24A1** | 3,619 | 5,938 | **▼ - match** | 0,105 |
| **GJC1** | 3,476 | 3,992 | **▼ - match** | 0,632 |
| **PPAP2B** | 6,064 | 4,916 | **▲ - match** | 0,387 |
| **ARHGAP29** | 2,579 | 2,606 | **▼ - match** | 0,980 |
| **TSPAN1** | 3,132 | 2,328 | **▲ - match** | 0,508 |
| **EHF** | 2,628 | 0,596 | **▲ - match** | 0,028 |
| **SEMA3A** | 8,421 | 7,052 | **▲ - match** | 0,461 |
| **KRT7** | 2,106 | -1,359 | **▲ - match** | 0,005 |
| **PRKACB** | -0,972 | 1,274 | **▼ - match** | 0,024 |
| **ANXA10** | 2,073 | 3,941 | **▼ - match** | 0,043 |
| **SERINC2** | -0,149 | -0,671 | **▲ - match** | 0,486 |
| **MID1** | 1,332 | 1,576 | **▼ - match** | 0,744 |

### Comparative Genomic Hybridization studies

This study was performed to verify any structural and numerical changes of chromosomes in CYC116 and ZM447439 resistant clones. Affymetrix Whole-genome 2.7M Arrays were used for this study. Amplifications or deletions for 140 genes among the disclosed gene list of certain chromosomal regions were found. Amplifications and deletions reflect the gene expression changes and thus can be used for diagnostics of patients resistant to Aurora kinase inhibitors.

### Proteomic studies

Two clones from each group were selected to determine differential protein level in comparison to controls. Lysates were prepared in four independent replicates for 2DE electrophoresis and subsequent protein identification by mass spectrometry. Two pH gradients were employed during isoelectric focusing including 4-7 and 6-11 to separate the proteins in the first dimension. Differentially expressed proteins were identified by MALDI-TOF/TOF. In ZM447439 resistant clones (R3.1: p53+/+, R3.2: p53+/+, R4.2: p53-/-, and R4.3: p53-/-), 77 protein candidates displayed differential expression. In CYC116 clones (R1.2: p53+/+, R1.3: p53+/+, R2.1: p53-/-, R2.2: p53-/-), 73 protein candidates displayed differential expression. Differential spots having fold-change >1.2 and p-value <0.05 (ANOVA) were considered as significant in proteomic analysis.

### EXAMPLE 2:

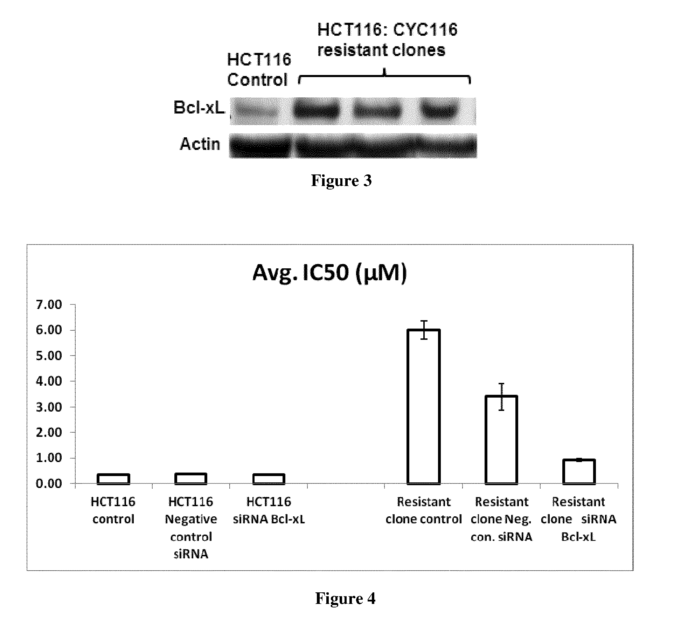

Microarray based gene expression analysis revealed up-regulation of Bcl-xL (BCL2L1) in HCT116 p53+/+ and HCT116 p53-/- resistant clones towards CYC116. Up-regulation of Bcl-xL in CYC116 resistant clones was statistically significant (p <0.001) and ~2 fold. Significant up-regulation of Bcl-xL in CYC116 resistant clones formed a strong rationale to test ABT-263 and anti-Bcl-xL siRNA in cell proliferation assay. In addition to RNA level, Bcl-xL upregulation was also confirmed at protein level by western blotting (Fig. 3)

### MTT based cell proliferation assay

This method is performed based on the principle that viable cells can reduce yellow colored MTT (3-(4, 5-dimethylthiazol-2-yl)-2, 5-diphenyltetrazolium bromide) salt to purple colored formazan. The intensity of the purple colored produced is directly proportional to number of viable cells, which can be measured calorimetrically. To determine the half inhibitory concentration (IC50) of any medicinal agent MTT assay is very reliable and well accepted. To determine the ABT-263's IC50 value, 3000 cells in 80 µl of cultivation medium were seeded in 96 well plates. 20 µl of each concentration of ABT-263 (prepared by serial-dilution 1:3, 10 µM top concentration - 0.01 µM lowest concentration) of the compound prepared in five-fold concentration stocks, were added to cells. The assay was carried out in 2 technical replicates for each concentration and in 3 biological replicates. Alongside blank and controls were included and incubated for 96 hrs. At the end of the assay time point 10 µl of MTT/well (Sigma) (10 mg/ml) was added and incubated until the appearance of violet formazan crystals. The formazan was dissolved with 100 µl/well 10 % aqueous SDS (pH: 5.5) by incubating the plates at 37 °C overnight. The optical density was measured at 540 nm using the Labsystem IMS reader and the IC50 values were determined using Chemorezist software.

We tested ABT-263 activity on two clones from each group of resistant clones. Particularly polyploid HCT116: CYC116 resistant clones with wild type p53 became highly sensitive (Average: 11 fold) to ABT-263 than HCT116 p53+/+ parent cell line (Fig. 2). This sensitivity corresponds to overexpression of Bcl-xL in HCT116: CYC116 resistant clones, determined at protein level (Fig. 3).

To validate the role of Bcl-xL overexpression in CYC116 induced resistance, we also genetically downregulated Bcl-xL using RNA interference. Knockdown of Bcl-xL, followed by CYC116 treatment significantly sensitized resistant tumors to CYC116 (Fig. 4). The IC₅₀ value of CYC116 on one HCT116 p53+/+: CYC116 resistant clone (used in siRNA study) is 6 µM, which is 18 fold higher than HCT116 parent cell line (0.34 µM). Knockdown of Bcl-xL followed by CYC116 treatment sensitized this resistant clone (0.9 µM) close to parent cell line. Knockdown of Bcl-xL in HCT116 parent cell line (low Bcl-xL expression) has no sensitization effect towards CYC116 (Fig. 4). This confirms the involvement of antiapoptotic Bcl-xL in CYC116 induced resistance. Inhibition of Bcl-xL either pharmacologically or genetically is advantageous to restore CYC116 sensitivity selectively in resistant clones that overexpress Bcl-xL. On the other hand polyploid HCT116 p53-/-: CYC116 resistant clones displayed significant cross-resistance to ABT-263 compared to parent HCT116 p53-/- cells. Both p53+/+ and p53-/- diploid ZM447439 resistant clones were resistant to ABT-263. These findings confirm that polyploid genotype induced by CYC116 is highly vulnerable to ABT-263 in the presence of wild type p53. Hence CYC116 induced phenotype can be exploited in the clinic by combining ABT-263 to overcome the resistance or even prevent emergence of resistance.

### Western Blot analysis

Cellular lysates were prepared using RIPA buffer (pH 8.0, 150 mM NaCI, 50 mM Tris-Cl, 1% NP-40, 0.1 % SDS, 0.5% deoxycholic acid). Proteins were separated using 8% SDS-PAGE gel and transferred to nitrocellulose membrane. The membrane was blocked in PBC containing 5% non-fat dry milk powder and 0.05% Tween20. The primary antibodies were prepared in blocking solution and the membrane was incubated overnight. After washing, the membrane was incubated in secondary antibody for 1 hour. The chemiluminescent signal was detected using ECL plus reagent.

### Bcl-xL knockdown by siRNA Transfection

0.1 x 10⁶ cells were seeded in 6 well plates in 2 ml of media. The cells were incubated for 24 h prior to the addition of Bcl-xL siRNA. The cells were washed with PBS and added 2 ml of fresh media without antibiotics. Bcl-xL siRNA and negative control siRNA purchased from Origene were diluted in RNase-free duplex buffer to get 10 µM concentration. The diluted siRNA was heated at 94 °C for 2 minutes for the formation of duplexes. 2.2 µl of diluted siRNA was added to 200 µl of jetPRIME buffer (Polyplus transfection), followed by the addition of 4 µl of jetPRIME transfection reagent, mixed and allowed to incubate for 15 minutes at room temperature. This mixture was added drop by drop to 2 ml of media, there by the final concentration of siRNA was 10 nM. The plates were incubated for 24 h, removed the media and added fresh media without siRNA. The cellular lysates were prepared at 72 hours and 96 hours to determine the Bcl-xL downregulation by western blotting. Particularly with two types of siRNAs downregulation of Bcl-xL was persisted until 96 hours. Negative control siRNA and transfection reagent has no effect on Bcl-xL expression. To prove the importance of Bcl-xL in induction of drug resistance to Aurora kinase inhibitors genetically, one Bcl-xL highly overexpressing p53 wild type CYC116 resistant clone was used for optimization. Cells which were transfected with anti-Bcl-xL siRNAs for 24 h were used for MTT assay to determine efficacy of Bcl-xL knockdown and CYC116 combination in comparison to CYC116 alone or control siRNA. Data clearly shows that genetic inhibition of Bcl-xL expression restores sensitivity of CYC116 resistant cells to the inhibitor.

### Industrial Applicability

The genes and proteins identified in the present invention can be used to monitor response to Aurora kinase inhibitors in clinical setting, to monitor the efficacy of Aurora kinase inhibitors therapy, to stratify patients according to the expression of these genes, etc. AstraZeneca's AZD1152 (Aurora B specific) is currently in phase II clinical trials. Both ZM44739 and AZD1152 have nearly identical mode of actions in cancer cells. ZM447439 and CYC116 resistant clones were highly cross-resistant (Table 1) to AZD1152 (AstraZeneca's Aurora B specific inhibitor), MLN8054 (Millennium's Aurora A specific inhibitor), and VX-680 (Vertex's pan-Aurora inhibitor). This strongly indicates similar mechanisms of tumor cell resistance towards these compounds. Hence the ZM447439 gene expression data and proteomics data is suitable to use in predicting AZD1152 long-term response. CYC116 data can also be used to predict AZD1152 and other Aurora kinase inhibitors response based on the fact that CYC116 clones are highly cross-resistant to AZD1152, VX-680, and MLN8054.
By the use of the prediction of sensitivity of patients to Aurora kinase inhibitors, the therapy can be administered only to those patients for whom it is beneficial, thereby decreasing the overall costs of cancer therapy and side effects. Those patients for whom the Aurora kinase inhibitors therapy would not bring any benefit, can be quickly selected for another therapy with medicaments which are more suitable for them and do not need to undergo an unnecessary and ineffective treatment. Moreover, the genes and their pathways identified in this invention as hallmarks of Aurora kinase drug resistance can be used as future therapeutic targets to develop novel strategies for overcoming the drug resistance
Also, the present invention provides for the use of a Bcl-2 family of inhibitors in combination with an Aurora kinase inhibitors for use in the treatment of Aurora kinase inhibitor-resistant tumors in order to overcome the resistance.

## Claims

1. A method for determining the sensitivity of a patient suffering from a cancer disease to Aurora kinase inhibitor therapy, which comprises determining *in vitro* in the cancer cells or body fluids taken from the patient the expression of at least one gene selected from the group comprising:
| **Gene** | **Change in expression determining resistance** |
|---|---|
| PBX1 | increase |
| **MID1** | **decrease** |
| **EHF** | **increase** |
| ALDH3A1 | increase |
| SSFA2 | decrease |
| SEPT2 | decrease |
| PVRL3 | decrease |
| SYTL2 | increase |
| KLK7 | increase |
| **ARHGAP29** | **decrease** |
| **A4GALT** | **increase** |
| APOBEC3H | increase |
| OAS1 | increase |
| 8084630 | increase |
| FXYD3 | increase |
| TSPAN5 | decrease |
| **CYP1A1** | **increase** |
| **PRKACB** | **decrease** |
| AVPI1 | increase |
| IGF2BP3 | decrease |
| **GJC1** | **decrease** |
| NRP2 | increase |
| HAS2 | increase |
| SCG2 | decrease |
| AQP3 | increase |
| FRMD5 | decrease |
| IFI44 | increase |
| SPRY4 | decrease |
| RNF125 | increase |
| ZFP36L1 | increase |
| AREG | increase |
| PRSS22 | increase |
| FNTA | decrease |
| ABCC2 | decrease |
| SERINC5 | increase |
| NEK10 | increase |
| NOV | increase |
| GRHL3 | increase |
| NEK3 | decrease |
| KLK8 | increase |
| ELOVL6 | decrease |
| 8062284 | increase |
| FYTTD1 | decrease |
| PRKCQ | increase |
| ATP9A | increase |
| DFNA5 | decrease |
| PTK6 | increase |
| SYK | increase |
| ALDH1A3 | increase |
| APOBEC3F | increase |
| CYP4F12 | increase |
| MAML2 | increase |
| SLC37A2 | increase |
| PAAF1 | increase |
| NEBL | decrease |
| CYP4F3 | increase |
| GNG5 | decrease |
| KLK6 | increase |
| ITGB7 | increase |
| NHS | increase |
| ATP13A3 | increase |
| SLC2A1 | increase |
| INTS10 | decrease |
| HOXA2 | increase |
| ANKH | increase |
| SOX4 | decrease |
| MFI2 | increase |
| HOXB9 | increase |
| KLK10 | increase |
| KRTAP3 | increase |
| C21orf63 | increase |
| APOBEC3C | increase |
| FAM49A | increase |
| TRAF3IP1 | decrease |
| S100A14 | decrease |
| C3orf57 | increase |
| LTBP3 | increase |
| CTSC | increase |
| LOXL4 | increase |
| HAS3 | increase |
| TRIM16L | decrease |
| PDE7A | decrease |
| RAB27B | increase |
| IL13RA2 | increase |
| ETS2 | decrease |
| RPL30 | decrease |
| CR2 | increase |
| LPIN1 | decrease |
| **BCL2L1** | **increase** |
| PERP | increase |
| HDAC2 | decrease |
| PORCN | increase |
| SECTM1 | increase |
| HSP90AB3P | decrease |
| HSP90AB1 | decrease |
| RPP30 | decrease |
| PKIB | decrease |
| IGFBP6 | increase |
| SAMD13 | decrease |
| MAL2 | decrease |
| SQLE | decrease |
| CD33 | increase |
| ZNF84 | decrease |
| WLS | increase |
| SYTL5 | decrease |
| SLC7A8 | increase |
| PPFIBP1 | decrease |
| ZNF493 | decrease |
| SLC5A1 | increase |
| STXBP6 | decrease |
| ZNF675 | decrease |
| 8099393 | decrease |
| BAMBI | increase |
| AMOTL1 | decrease |
| CLU | decrease |
| ZNF26 | decrease |
| ZNF91 | decrease |
| ZNF266 | decrease |
| IL18 | decrease |
| **FAM122B** | **decrease** |
| DOCK5 | decrease |
| SLC04A1 | increase |
| SNORD5 | decrease |
| SNORA18 | decrease |
| MIR1304 | decrease |
| ILF2 | decrease |
| ATP6AP1 | increase |
| MEF2C | decrease |
| C5orf13 | increase |
| EXOSC9 | decrease |
| ALDH2 | increase |
| FUT8 | decrease |
| CDA | increase |
| TOX2 | increase |
| FGF9 | increase |
| OAS3 | increase |
| SEMA3D | increase |
| MIR15A | decrease |
| DLEU2 | decrease |
| MIR16-1 | decrease |
| USP22 | increase |
| **INPP4B** | **decrease** |
| TNS4 | increase |
| MNS1 | decrease |
| 7893924 | increase |
| TCF21 | decrease |
| ZBED2 | decrease |
| C1 DP1 | decrease |
| **BDNF** | **decrease** |
| **PPAP2B** | **increase** |
| 7894891 | increase |
| CDC23 | decrease |
| 8109424 | increase |
| SMNDC1 | decrease |
| SART3 | decrease |
| DDX5 | decrease |
| MMP14 | decrease |
| FANCL | decrease |
| 8098287 | decrease |
| TARDBP | decrease |
| CASP4 | increase |
| SNORD22 | decrease |
| SNORD28 | decrease |
| SNORD29 | decrease |
| SNORD30 | decrease |
| **ERI1** | **decrease** |
| RPSA | decrease |
| CPOX | decrease |
| 7894781 | decrease |
| PALLD | decrease |
| MKX | decrease |
| CSMD3 | increase |
| ENC1 | decrease |
| **SERINC2** | **increase** |
| CID | decrease |
| CAV1 | decrease |
| AKT3 | increase |
| **ANXA10** | **decrease** |
| KLRC2 | decrease |
| **CYP24A1** | **decrease** |
| WNT16 | decrease |
| 8148309 | decrease |
| **CAMK2D** | **decrease** |
| RHOBTB3 | decrease |
| PDE4B | decrease |
| **HTR7** | **decrease** |
| COL12A1 | decrease |
| TIAM1 | decrease |
| KLRC3 | decrease |
| KRT23 | decrease |
| ZNF280A | decrease |
| **TBX3** | **increase** |
| UNC13A | increase |
| RUNX2 | increase |
| TRIB2 | increase |
| **TSPAN1** | **increase** |
| ARMC4 | decrease |
| MPP7 | decrease |
| **KRT7** | **increase** |
and/or determining *in vitro* in the cancer cells or body fluids taken from the patient the level of at least one protein selected from the group comprising:
| **Protein Name** | **Change in level determining resistance** |
|---|---|
| Chloride intracellular channel protein 1 | Decrease |
| Isocitrate dehydrogenase [NAD] subunit alpha, mitochondrial | Decrease |
| Keratin, type II cytoskeletal 18 | Decrease |
| Keratin, type I cytoskeletal 19 | Decrease |
| Rab GDP dissociation inhibitor beta | Decrease |
| Splicing factor, arginine/serine-rich 7 | Decrease |
| Platelet-activating factor acetylhydrolase IB subunit beta | Decrease |
| Serpin B5 | Increase |
| Ras GTPase-activating protein-binding protein 1 | Increase |
| Ubiquitin carboxyl-terminal hydrolase isozyme L3 | Increase |
| Phosphoserine phosphatase | Increase |
| 78 kDa glucose-regulated protein | Decrease |
| Elongation factor 1-delta | Decrease |
| Heat shock cognate 71 kDa protein | Increase |
| Phosphoglycerate mutase 1 | Increase |
| GTP-binding nuclear protein Ran | Increase |
| Fascin | Increase |
| Proteasome subunit beta type-2 | Increase |
| Heterogeneous nuclear ribonucleoprotein H | Decrease |
| Phosphoserine aminotransferase | Increase |
| Eukaryotic translation initiation factor 4H | Increase |
| Annexin A3 | Increase |
| Tropomyosin alpha-4 chain | Decrease |
| Gamma-enolase | Increase |
| Splicing factor, arginine/serine-rich 7 | Decrease |
| Serpin B5 | Increase |
| Heterogeneous nuclear ribonucleoprotein G | Decrease |
| Heat shock protein HSP 90-beta | Increase |
| dCTP pyrophosphatase 1 | Decrease |
| Inositol-3-phosphate synthase 1 | Increase |
| Nucleophosmin | Increase |
| Ras-related protein Rab-1 B | Increase |
| Heat shock cognate 71 kDa protein | Increase |
| Eukaryotic translation initiation factor 3 subunit G | Increase |
| Inosine triphosphate pyrophosphatase | Increase |
| Heat shock protein HSP 90-alpha | Decrease |
| Calretinin | Increase |
| Serine/arginine-rich splicing factor 2 | Decrease |
| Heterogeneous nuclear ribonucleoprotein L | Decrease |
| Heterogeneous nuclear ribonucleoprotein H3 | Decrease |
| Pyruvate kinase isozymes M1/M2 | Increase |
| 6-phosphofructokinase type C | Decrease |
| Voltage-dependent anion-selective channel protein 2 | Increase |
| Voltage-dependent anion-selective channel protein 1 | Increase |
| Serine hydroxymethyltransferase, mitochondrial | Increase |
| Phosphoserine aminotransferase | Increase |
| Malate dehydrogenase, mitochondrial | Increase |

2. The method according to claim 1, wherein the expression of at least two, three, four, five, six, seven, eight, nine or ten genes and/or the level of at least two, three, four, five, six, seven, eight, nine or ten proteins is determined.

3. The method according to claim 1, wherein the expression of at least one gene selected from the group comprising MIDI, EHF, ARHGAP29, A4GALT, CYP1A1, PRKACB, GJC1, BCL2L1, FAM122B, INPP4B, BDNF, PPAP2B, ERI1, SERINC2, ANXA10, CYP24A1, CAMK2D, HTR7, TBX3, TSPAN1 and KRT7 is determined.

4. The method according to claim 3, wherein the expression of at least two, three, four, five, six, seven, eight, nine or ten genes is determined.

5. The method according to claim 3, wherein the expression of all genes MIDI, EHF, ARHGAP29, A4GALT, CYP1A1, PRKACB, GJC1, BCL2L1, FAM122B, INPP4B, BDNF, PPAP2B, ERI1, SERINC2, ANXA10, CYP24A1, CAMK2D, HTR7, TBX3, TSPAN1, KRT7 is determined.

6. The method according to claim 1, wherein the expression of at least one gene selected from the group comprising CYP24A1, EHF, KRT7, PRKACB and ANXA10 is determined.

7. The method according to claim 6, wherein the expression of at least two, three, four or five genes is determined.

8. The method according to claim 6, wherein the expression of all genes CYP24A1, EHF, KRT7, PRKACB and ANXA10 is determined.

9. The method according to any of the preceding claims, wherein the Aurora kinase inhibitor is preferably selected from CYC 116 (4-methyl-5-(2-(4-morpholinophenylamino)pyrimidin-4-yl)thiazol-2-amine), ZM447439 (N-[4-[[6-Methoxy-7-[3-(4-morpholinyl)propoxy]-4-quinazolinyl]amino]phenyl]benzamide), AZD1152 (2-[ethyl-[3-[4-[[5-[2-(3-fluoroanilino)-2-oxoethyl]-1Hpyrazol3yl]amino]quinazolin7-yl]oxypropyl]amino]ethyl dihydrogen phosphate), VX-680 (N-[4-[4-(4-methylpiperazin-1-yl)-6-[(5-methyl-1H-pyrazol-3-yl)amino]pyrimidin-2-yl]sulfanylphenyl]cyclopropanecarboxamide), MLN8054 (4-[[9-chloro-7-(2,6-difluorophenyl)-5H-pyrimido[5, 4-d][2]benzazepin-2-yl]amino]benzoic acid), PHA-739358 (N-[5-[(2R)-2-methoxy-2-phenylacetyl]-4,6-dihydro-1H-pyrrolo[3, 4-c]pyrazol-3-yl]-4-(4-methylpiperazin-1-yl)benzamide), MLN8237 (4-[[9-chloro-7-(2-fluoro-6-methoxyphenyl)-5H-pyrimido[5,4-d][2]benzazepin-2-yl]amino]-2-methoxybenzoic acid), AT-9283 (1-cyclopropyl-3-[(3Z)-3-[5-(morpholin-4-ylmethyl)benzimidazol-2-ylidene] -1,2-dihydropyrazol-4-yl]urea).

10. The method according to any of the preceding claims, wherein the cancer disease is selected from the group comprising sarcomas, colorectal, melanoma, skin, breast, thyroid, glioblastoma, lung, prostate, ovarian, cervical, uterine, head and neck, hematological, gastric, oesophageal, neural, pancreatic, and renal cancers.

11. A Bcl-2 family inhibitor in combination with an Aurora kinase inhibitor for use in the treatment of Aurora kinase inhibitor-resistant tumors.
